# EUROPEAN PATENT SPECIFICATION

(11) **EP 1 376 129 B1**
(45) Date of publication and mention of the grant of the patent: **10.10.2007**
(21) Application number: 03014736.7
(22) Date of filing: 27.06.2003
(51) Int. Cl.: G01N 33/543

(54) **Magnetic carrier for biological substance, production method thereof and isolation method of biological substance using the same**
Magnetischer Träger für biologische Substanzen, Verfahren zur seiner Produktion und seiner Verwendung zur Isolierung dieser biologischen Substanzen
Supports magnétiques pour substances biologiques, leur production et leur utilisation pour isoler ces substances

(30) Priority: 27.06.2002 JP 2002188140; 07.08.2002 JP 2002230533; 12.09.2002 JP 2002267170
(43) Date of publication of application: 02.01.2004
(73) Proprietor: Toyo Boseki Kabushiki Kaisha, Osaka-shi, Osaka 530-8230 (JP); HITACHI MAXELL, LTD., Ibaraki-shi, Osaka 567-8567 (JP)
(72) Inventor: Nishiya, Yoshiaki, c/o Toyo Boseki K. K., Osaka-shi, Osaka 530-8230 (JP); Tsuboi, Satoko, Satsukiryo, Hitachi Maxell, Ltd., Otokuni-gun, Kyoto 618-0071 (JP); Kishimoto, Mikio, Moriya-shi, Ibaraki 302-0102 (JP)
(74) Representative: Vossius & Partner

(56) References cited:
- EP-A- 0 343 934
- EP-A- 0 450 092
- EP-A- 0 531 990
- EP-A- 0 541 113
- EP-A- 1 154 443
- EP-A- 1 335 003
- WO-A-00/32762
- WO-A-01/14591
- US-A1- 2002 000 398
- US-B1- 6 255 477

## Description

The present invention relates to a magnetic carrier, a production method thereof and a method of isolating biological substance using the carrier.

Various methods of isolating an objective biological substance from a' sample containing a biological substance, such as nucleic acid, protein, carbohydrate and the like, have been conventionally studied. As such methods, methods comprising extraction and separation using organic solvents, methods of isolating the objective substance using a molecule filter based on the size of the molecule, isolation methods utilizing a carrier capable of reversibly binding with a particular biological substance, and the like are known. Of the above-mentioned methods, isolation methods utilizing a carrier have many advantages for simultaneous processing of a number of samples. Particularly, isolation utilizing a magnetic carrier is highly convenient. This is because a biological substance - magnetic carrier complex can be collected by applying a magnetic field, and a collecting device, such as centrifuge and the like, is not required.

Methods of isolating biological substance utilizing a magnetic carrier have been specifically developed as isolation methods of various nucleic acids. As a magnetic carrier for the isolation of nucleic acid, magnetic responsive particles are known, which contain iron oxide having a coating of polymerizable silane capable of covalent binding with affinity molecules (e.g., nucleic acid and the like) (JP-A-60-1564). Magnetic responsive particles require a silane coating that binds with affinity molecules (e.g., nucleic acid). As an improvement of the above, spherical magnetic silica particles containing superparamagnetic metal oxide are known (JP-A-9-19292 and JP-A-2001-78761) Such magnetic silica particles are complexes of superparamagnetic metal oxide with inorganic porous wall substance consisting of fine silica particles and have a specific surface area of 100-800 m²/g. Preferable superparamagnetic metal oxide content is 10-60 wt%, and preferable particle size is 0.5-15 µm.

In addition, magnetic silica particles having a structure, in which plural fine core particles comprising metal or metal oxide consisting of multiple magnetic domains are coated with a film or fine particles of silicon oxide, have been also proposed (JP-A-2000-256388).

Moreover, an analysis method and a device using biological substances bound with a substance having magnetic reactivity are also known (WO86/05815). According to this method, a magnetized particle or a magnetizable particle coated with a substance capable of binding with a single strand nucleic acid is used to separate and detect a single strand nucleic acid. Specifically, nitrocellulose, which is one kind of cellulose derivative, is coated on the surface of a magnetized particle, and nitrocellulose and single strand nucleic acid of DNA or RNA are specifically bound with each other. According to this method, since single strand nucleic acid of DNA or RNA is specifically bound with the magnetic carrier, the surface of the magnetized particle needs to be covered with a cellulose derivative such as nitrocellulose and the like.

The use of a polycationic support for the purification and isolation of a biological substance, particularly, use of a polycationic support for the purification, isolation and hybridization of nucleic acid, is also known (Japanese Patent Application under PCT laid-open under kohyo No. hei-1-502319). As a support, metal oxide, glass, polyamide and the like are recited as examples. As a polycationic magnetic responsive particle, a magnetized amine microsphere having a particle size of about 1 µm (magnetic microsphere) and the like can be used. The binding of nucleic acid with a support is considered to be based on the ionic bond between a magnetized amine microsphere having a positive charge and a sugar phosphate main chain of nucleic acid, which has a negative charge.

In addition, an isolation method of a pure biological material is also known, which uses a magnetic responsive particle comprising an internal core polymer particle and a magnetically responsive metal oxide/polymer coating uniformly covering the particle (Japanese Patent Application under PCT laid-open under kohyo No. hei-2-501753).

However, since a functional group is often added to the surface of the magnetized particle, these are advantageous for the isolation of nucleic acid based on specific adsorption but inconvenient for non-specific isolation.

In general terms, when a surface-coated magnetic particle is used as a solid phase carrier for the isolation of biological substance, large particles (e.g., diameter of not less than 20 µm) are advantageous since they tend to easily respond even in a weak magnetic field. On the other hand, large particles rapidly produce sedimentation, are poor in operability, have smaller specific surface area, and show low binding efficiency with biological substances. In contrast, smaller particles (e.g., diameter of not more than 0.1 µm) have a larger specific surface area, which in turn improves binding efficiency with nucleic acid and the like, and operability because sedimentation does not occur easily. On the other hand, since smaller particles have lower responsiveness to a magnetic field, a strong magnetic field is necessary to collect the carrier.

As described above, known isolation methods of biological substances using magnetic carriers still contain many aspects to be improved. Among others, dispersibility of the magnetic carrier in an aqueous solution of at sample containing the objective biological substance is directly related to the frequency of contact with the biological substance, which is an important factor responsible for the binding efficiency of the magnetic carrier and the objective biological substance, and further, separation efficiency of the biological substance. In addition, the collectability of the magnetic carrier due to the magnetic field directly affects efficiency of solid liquid separation and time saving in the isolation method of biological substances utilizing the magnetic field separation techniques.

WO 00/32762 discloses magnetic particles for purifying nucleic acids having a glass surface and a magnetic core. The core may have a size of 0.5 to 1.5 µm.

The present invention aims at solving the above-mentioned problems. The present invention mainly aims at providing a magnetic carrier superior in dispersibility in aqueous solutions as well as in the collectability due to the magnetic field, superior in reversible binding ability with a biological substance and elution property of the bound biological substance, as compared to conventional ones, and achieving improved isolation and purification efficiency of biological substance.

The present invention also aims at providing a magnetic carrier suitable for a micro TAS (Total Analysis System) which is a convenient method for analysis of nucleic acid comprising extraction and purification. The magnetic carrier to be applied to the micro TAS is desired to have a smaller particle size and to be superior in flowability in a solvent. It facilitates passage in the system by the magnetic field. For this end, it is desirable to coat magnetic particles with a compound for nucleic acid binding, which is superior in uniform adhesion.

To achieve the above-mentioned obj ect, the present inventors have studied in detail the correlation between magnetic property of magnetic carriers and nucleic acid isolation efficiency, which has never been considered in the field of biological substances, particularly nucleic acid purification. As a result, they have found that, for a magnetic carrier containing a ferromagnetic iron oxide particle to have binding property of biological substances, collectability due to the magnetic field, dispersibility in aqueous solution and elution property of biological substance in combination, control of the magnetic property of the magnetic carrier is extremely important. To be specific, a magnetic carrier having, of the magnetic properties, a coercive force and a saturation magnetization in particular ranges, and preferably an average particle size in a particular range, can solve the above-mentioned problems.

In addition, the magnetic carrier capable of solving the above-mentioned problems may be coated with a compound containing a ferromagnetic iron oxide particle and silicon. Of such magnetic carriers, a magnetic carrier wherein a specific amount of silica is coated on the surface of a spherical or granular ferromagnetic iron oxide particle have well balanced bindability with biological substances (particularly nucleic acid), collectability due to the magnetic field, dispersibility in aqueous solution and elution property of biological substance. In addition, a magnetic carrier wherein a compound containing silicon and aluminum is coated on the surface of a ferromagnetic iron oxide particle shows fine coating uniformity, thereby solving the above-mentioned problems, and is superior in flowability.

The present invention is defined by the features of the claims.
Fig. 1 shows an SEM image (magnification : x30,000) of the magnetic carrier according to the present invention obtained in Example 1.
Fig. 2 shows an SEM image (magnification : x30,000) of the magnetic carrier according to the present invention obtained in Example 5.
Fig. 3 shows an SEM image (magnification: x30,000) of the magnetic carrier obtained in Reference Example 13.
Fig. 4 shows electrophoresis images of the nucleic acids recovered using the magnetic carriers obtained in Reference Examples 13-15 and Comparative Reference Example 6, wherein

M means a molecular weight marker;
lanes 1 and 2 show the results of nucleic acid extraction purification by the use of the carrier of Reference Example 13;
lanes 3 and 4 show the results of nucleic acid extraction purification by the use of the carrier of Reference Example 14;
lanes 5 and 6 show the results of nucleic acid extraction purification by the use of the carrier of Reference Example 15; and
lanes 7 and 8 show the results of nucleic acid extraction purification by the use of the carrier of Comparative Reference Example 6.

The magnetic carrier to be used in the present invention has [1] a saturation magnetization of 30-80 A·m²/kg, [2] a coercive force of 2.39-11.94 kA/m. and [3] an average particle size of 0.12-0.45 µm.

The magnetic carrier further comprises magnetite particles and a silica coating on the surface of said magnetite particles, wherein the amount of the silica coating is 3-100 wt% of the magnetite particles. All claimed methods and uses involve said carrier.

### [1] Saturation magnetization

The saturation magnetization of the magnetic carrier is 30--80 A·m²/kg (30-80 emu/g) more preferably 35-75 A·m²/kg (35-75 emu/g).

The saturation magnetization relates to the collectability of magnetic carrier. In general, a greater saturation magnetization is associated with more improved responsiveness to the magnetic field. As a result, magnetic carrier in a suspension can be collected efficiently in short time. As long as the saturation magnetization is within the above-mentioned range, the collection performance of the magnetic carrier can be most improved without impairing the bindability to a biological substance to be mentioned below. When the saturation magnetization is less than 10 A·m²/kg (10. emu/g), the responsiveness of the magnetic carrier to the magnetic field is degraded to make flocculation difficult (difficult collection). For a magnetic carrier having a saturation magnetization exceeding 80 A·m²/kg (80 emu/g) to be obtained, the amount of a biologically binding substance (silica etc.) to be coated on the magnetic particle needs to be decreased, which in leads to the propensity toward degraded biding performance with a biological substance and lower dispersibility.

The saturation magnetization of the magnetic carrier can be determined by, for example, measuring the magnetization upon application of 796.5 kA·m (10 kilooersted) magnetic field using a vibrating sample magnetometer (TOEI INDUSTRY CO., LTD).

When the magnetic carrier is a ferromagnetic iron oxide particle coated with silica, the saturation magnetization of the magnetic carrier is determined by the saturation magnetization of the ferromagnetic iron oxide particle and the amount of the silica to be coated. In this case, the saturation magnetization of the magnetic carrier is in the range of 30-80 A·m²/kg (30-80 emu/g).

When the magnetic carrier comprises a ferromagnetic iron oxide particle coated with a compound comprising silicon and aluminum, the saturation magnetization of the magnetic carrier is determined by the saturation magnetisation of the ferromagnetic iron oxide particle and the amount of the compound comprising silicon and aluminum. In this case, the saturation magnetisation of the magnetic carrier is most preferably in the range of 10-80 A·m²/kg (10-80 emu/g)*.* However, such a carrier is not claimed.

### [2] Coercive force

The coercive force of the magnetic carrier is 2.39-11.94 kA/m (30-150 oersted, more preferably 3.19-10.35 kA/m (40-130 oersted).

The coercive force is closely related to the elution property of the biological substance from the magnetic carrier. The magnetic carrier is magnetized to some degree by the magnetic field applied for collection. In this case, a greater coercive force causes a greater flocculation force between magnetic carriers which in turn degrades dispersibility of the magnetic carrier during elution of biological substance from the magnetic carrier. As a result, elution of the biological substance bound with the magnetic carrier in the solution becomes difficult and the isolation efficiency of the biological substance tends to become lower. The coercive force of the magnetic carrier does not cause any practical problem while it is small, but production of a magnetic carrier, showing a small coercive force is associated with restrictions on the kind of materials (ferromagnetic iron oxide particle etc.) to be used and synthesis methods of the magnetic carrier. The present inventors have studied the optimal range of coercive force that does not influence the biological substance, particularly purification of nucleic acid, and found that no practical problem occurs when the coercive force is within the above-mentioned range.

The coercive force of the magnetic carrier can be determined using a vibrating sample magnetometer (TOEI INDUSTRY CO., LTD) by, for example, applying a magnetic field of 796.5 kA/m (10 kilooersted) for saturation magnetization, reducing the magnetic field to nil, applying the magnetic field such that the magnetic field gradually increases in the reverse direction and reading the intensity of the applied magnetic field at the time when the magnetization value becomes nil.

When the magnetic carrier is a ferromagnetic iron oxide particle coated with silica, the coercive force of the magnetic carrier is mostly determined by the coercive force of the ferromagnetic iron oxide particle. In this case, the coercive force of the magnetic carrier is 2.39-11.94 kA/m (30-150 oersted) and the saturation magnetization is 30-80 A·m²/kg (30-80 emu/g).

### [3] Average particle size

The magnetic carrier an average particle size of 0.12-0.45 µm. When the average particle size of the magnetic carrier is too small (e.g. : less than 0.1 µm), collection of magnetic carrier by the magnetic field becomes difficult, and re-dispersion upon removal of the magnetic field tends to become difficult. When the average particle size of the magnetic carrier is too large (e.g.: above 10 µm), the magnetic carrier easily forms sedimentation and the specific surface area becomes too small, which often degrades the binding performance with a biological substance.

As used herein, the "particle size" of the magnetic carrier refers to the maximum length of all the lengths in any direction of the particle. In addition, the average particle size of the above-mentioned magnetic carrier is calculated by, for example, measuring the particle size of each of 300 particles on a transmission electron microscopic photograph and calculating the number average thereof.

The shape of the magnetic carrier is preferably spherical or granular.

When the magnetic carrier is a ferromagnetic iron oxide particle coated with silica, a spherical or granular particle has an average particle size of 0.12-0.45 µm. In this case, particles having a structure wherein plural ferromagnetic iron oxide particles are coated with silica, may be present, as long as the average particle size is within the above-mentioned range.

When the magnetic carrier is a ferromagnetic iron oxide particle coated with a compound comprising silicon and aluminum, an average particle size is preferably 0.1-10 µm, more preferably 0.12-8 µm. However, such a carrier is not claimed.

The magnetic carrier to be used for the present invention affords superior isolation efficiency by simultaneously satisfying the aforementioned [1], [2] and [3]. To be specific, the magnetic carrier of the present invention is strikingly superior in the capability of the following (a) to (c) as compared to conventional magnetic carriers:
(a) dispersing in an amount of at least 20 mg in 1 mL of an aqueous solution of a sample containing a biological substance,
(b) being collected by not less than 90 wt% within 3 seconds in the presence of a magnetic field of 2000-3000 gauss, and
(c) reversibly binding with at least 0.4 µg of a biological substance per 1 mg thereof.

In the following, (a) - (c) are explained.
(a) Dispersing in an amount of at least 20 mg in 1 mL of an aqueous solution of a sample containing a biological substance
   A superparamagnetic magnetic carrier, which is a typical magnetic carrier conventionally used in this field, is basically different from the magnetic carrier of the present invention (using ferromagnetic iron oxide particle and the like) in that it has a saturation magnetization of not more than 10 A·m²/kg, and a coercive force of not more than 0.80 kA/m. For the expression of a superparamagnetic magnetic carrier, a substance having very small saturation magnetization and coercive force is generally used, or should be made to have a small particle size (e.g.: not more than 0.1 µm).
   In general terms, a smaller particle size is conducive to fine dispersibility. As used herein, by the "disperse" is meant a state wherein magnetic carrier is stably floating in an aqueous solution of a sample containing a biological substance without forming a sedimentation in the aqueous solution upon visual observation. The dispersibility of such magnetic carrier can be evaluated by, for example, observation of the position of the interface between the sedimentation liquid and supernatant after allowing an aqueous solution containing the magnetic carrier to stand for a given time. When supernatant is not separated after standing for a long time, the dispersibility is evaluated to be fine. A magnetic carrier using a superparamagnetic particle having a small particle size (not more than 0.1 µm) generally shows fine dispersibility.
(b) being collected by not less than 90 wt% within 3 seconds in the presence of a magnetic field of 2000-3000 gauss
   Collection of a superparamagnetic magnetic carrier, which is a typical magnetic carrier conventionally used in this field, by 90 wt% in the presence of a 2000-3000 gauss magnetic field takes more than 10 seconds. With such conventional magnetic carrier, only about 1-50 wt% can be collected when placed in the presence of the above-mentioned magnetic field for 3 seconds. The easiness of collectability of magnetic carrier can be evaluated by, for example, leaving a solution containing the magnetic carrier on a magnet capable of generating a 2000-3000 gauss magnetic field for 3 seconds, removing the solution that was not collected by the magnetic field, and then measuring the weight of the magnetic carrier collected by the magnetic field. When a magnetic carrier having a saturation magnetization of not more than 10 A-m²/kg and a coercive force of not more than 0.80 kA/m is used, collection by 90 wt% in the presence of a 2000-3000 gauss magnetic field within 3 seconds is difficult, because the sensitivity to the magnetic field is low. In addition, collection of a magnetic carrier having a particle size of not more than 0.1 µm by 90 wt% within 3 seconds is difficult, because dispersibility in the solution is high and separation by the magnetic field is difficult.
   The 2000-3000 gauss external magnetic field is a range of a preferable external magnetic field for the separation of a complex of a magnetic carrier and a biological substance bound to each other from a sample, in the field of isolation of a biological sample containing a biological substance using a magnetic carrier. When it is less than 2000 gauss, the sensitivity of the complex to the magnetic field tends to become weak and when it exceeds 3000 gauss, an apparatus to produce the magnetic field becomes bulky and costly.
(c) reversible binding with at least 0.4 µg of a biological substance per 1 mg of carrier

A superparamagnetic magnetic carrier, which is a typical magnetic carrier conventionally used in this field, can reversibly bind with only about 0.3 µg of a biological substance per 1 mg of the carrier. As used herein, the capability of reversible binding means that a magnetic carrier and a biological substance can be artificially bound with each other and separated from each other by a reproductive method without changing the properties thereof. The capability of reversible binding can be confirmed by the method for isolating a biological substance from a sample containing the biological substance as described in the Examples below.

The above-mentioned (a) - (c) can be achieved by simultaneously satisfying the bindability with a biological substance, the collectability of magnetic carrier by a magnetic field, dispersibility of magnetic carrier and elution property of biological substance. Use of such magnetic carrier improves isolation and purification efficiency of biological substances.

The magnetic carrier comprises basically a ferromagnetic iron oxide particle (magnetite particle in the present invention) and silica to be coated on the ferromagnetic iron oxide particle. As used herein, by "coated" is meant that, a silica layer is formed on the outermost layer of the magnetic carrier, covering the outside of the ferromagnetic iron oxide particle, and is synominous with adhesion of silica to the vicinity of the surface of the ferromagnetic iron oxide particle. The silica layer may be formed to completely cover the ferromagnetic iron oxide particle. Alternatively, the ferromagnetic iron oxide particle may be partly exposed, as long as the bindability with silica and a biological substance to be isolated is not inhibited. Only one ferromagnetic iron oxide particle may be coated with silica to give one magnetic carrier, or a flock consisting of 2 to 100 ferromagnetic iron oxide particles may be coated with silica to give one magnetic carrier. The silica in the present invention includes SiO₂ crystal and other forms of silicon oxide molecules, skeleton of *Bacillariophyceae* consisting of SiO₂ and amorphous silicon oxide.

The ferromagnetic iron oxide particles having a spherical or granular shape are applied to a broad range of use such as those for magnetic recording, toner for copying machines, black color additive to various resins and the like. These ferromagnetic iron oxide particles are requested to uniformly disperse in a medium irrespective of a dry or wet method of use. Therefore, the ferromagnetic iron oxide particle undergoes a surface treatment to improve dispersibility. For the surface treatment, there are known a method comprising a surface treatment with an inorganic material, and a method comprising surface treatment with an organic material. As a method of surface treatment with an inorganic material, a coating of silica or alumina is generally formed.

Forming a silica coating near the surface of each ferromagnetic iron oxide particle is not particularly novel. However, when a silica coating is applied with the aim of improving the dispersibility for toner use and the like, forming of a uniform coating to cover the surface of each particle is important, and the amount of silica to be coated is not important. When improved dispersibility is desired, the amount of silica to be coated on the ferromagnetic iron oxide particle is generally several wt% at most. When improved dispersibility is desired, coating of, for example, not less than 2 wt% of silica does not result in further improvement in the dispersibility. Rather, the amount of redundant silica without magnetism increases, thereby decreasing saturation magnetization and blackness.

Therefore, there has been no use for a ferromagnetic iron oxide particle coated with several wt% or more of silica, which has obliterated the study of coating such large amount of silica.

The present inventors have prepared various magnetic carriers having varying amount of silica adhered to each ferromagnetic iron oxide particle and examined the bindability with nucleic acid, collection by magnetic field, dispersibility upon removal of magnetic field and elution property of nucleic acid, and unexpectedly found that a magnetic carrier coated with a far greater amount of silica than the amount of silica considered to be necessary for coating each ferromagnetic iron oxide particle, with the purpose of imparting dispersibility, shows superior performance not only in bindability with nucleic acid/collection by magnetic field, but also in dispersibility upon removal of magnetic field/elution property of nucleic acid.

That is, a magnetic carrier having an average particle size of 0.1-0.5 µm obtained by coating a spherical or granular ferromagnetic iron oxide particle with silica in an amount of 3-100 wt%, which is far greater than the amount conventionally considered to be optimal for imparting dispersibility, has dramatically high bindability with nucleic acid.

Due to the nature of nucleic acid showing preferential binding with silica, a greater adhesion amount of silica generally means a greater amount of binding with nucleic acid. In conventional magnetic carriers, however, a flock consisting of plural magnetic particles having a smaller particle size before coating with silica is coated with silica. Therefore, the average particle size of the resulting magnetic carrier becomes greater (0.5-15 µm), and the surface area effective for binding with nucleic acid becomes smaller.

In such magnetic carrier, adhesion of a large amount of silica only increases the film thickness of the silica layer on the surface, thus failing to substantially increase the surface area of the silica layer effective for binding with nucleic acid.

The present inventors have found that the amount of nucleic acid binding can be increased by directly adhering silica to ferromagnetic iron oxide particles and, for this to be achieved, the magnetic carrier comprising silica should be made smaller (see the above-mentioned [3]).

The "ferromagnetic iron oxide particle" in the present specification is a particle having magnetic responsiveness (sensitivity to the magnetic field), which can be obtained by oxidation of metal particles. The claims are limited to magnetic particles. The present inventors have examined the compatibility of various ferromagnetic iron oxide particles as a magnetic carrier for nucleic acid extraction through long years of experience in the development of magnetic material for magnetic recording. As the ferromagnetic iron oxide particle maghemite particle (γ-Fe₂O₃), magnetite particle (Fe₃O₄) (the present invention), manganese zinc ferrite particle (Mn₁₋ₓZnₓFe₂O₄), metal iron particle (α-Fe), iron-cobalt alloy particle (FeCo) and barium ferrite particle (BaFe₁₂O₁₉) are most suitable. Of these ferromagnetic iron oxide particles, iron oxide having a divalent iron ion, such as magnetite particle and the like, may be an intermediate iron oxide of magnetite and maghemite (magnetite-maghemite intermediate iron oxide particle), which is deviated from the above-mentioned stoichiometry to the extent the crystal structure can be maintained. Magnetic metal particles such as metal iron, iron-cobalt alloy particle and the like are unstable to moisture, and upon immersion into water, tend to show lower saturation magnetization. Manganese zinc ferrite particle tends to show lower saturation magnetization as compared to ferromagnetic iron oxide particle consisting only of iron. In addition, since barium ferrite particle has an excessively high coercive force, the magnetic carrier easily flocculates magnetically when eluting the bound nucleic acid after collection with a magnet and the like, thereby degrading the elution property of the nucleic acid. The above-mentioned "magnetic responsiveness" means that, when external magnetic field exists due to a magnet and the like, the particle shows sensitivity to the magnetic field, as evidenced by magnetization by the magnetic field, attraction by the magnet and the like.

The present inventors have coated various magnetic particles with silica and studied the compatibility as a magnetic carrier for isolation of biological substances to be mentioned below, and found that the aforementioned maghemite particle (γ-Fe₂O₃) and magnetite particle (Fe₃O₄), as well as an intermediate iron oxide particle thereof, are most suitable for achieving the above-mentioned saturation magnetization of the magnetic carrier and have superior balance in the properties. Particularly, since magnetite particle has a saturation magnetization about 15% greater than that of maghemite, particle and a lower coercive force, it is particularly preferable as a ferromagnetic iron oxide particle of the present invention.

The ferromagnetic iron oxide particle of the present invention is subject to no particular limitation as to its shape, and may have various shapes, such as sphere, ellipsoid, granular, plate, needle, polyhedral and the like. When it is needle or plate, an anisotropic coercive force may emerge. Thus, a spherical, granular or ellipsoidal shape free of such anisotropy is preferable, particularly preferably spherical. That is, the preferable aspect ratio (ratio of maximum length and minimum length as measured in any direction) of the particle is 0.5-2.0. As used herein, by the "spherical" is meant a shape having an aspect ratio of 1.0-1.2 (not less than 1.0 and not more than 1.2), and by the "ellipsoidal" is meant a shape wherein the aspect ratio exceeds 1.2 and is not more than 1.5. By the "granular" is meant one having the same length of particle in all directions, such as a sphere, and one free of particular anisotropy as a whole, though subject to change in length depending on the direction, such as ellipsoid having a greater length in only one direction.

While the ferromagnetic iron oxide particle content of the magnetic carrier in the present invention is limited by the claims, it is preferably 50-95 wt%, more preferably 60-90 wt%, of the magnetic carrier. When the content is less than 50 wt%, the saturation magnetization of the magnetic carrier becomes small; and the sensitivity to the magnetic field tends to be low. When the content exceeds 95 wt%, the amount of silica and the like to coat the ferromagnetic iron oxide particle becomes small, making isolation of nucleic acid difficult.

In the following, a preferable method is explained in detail while referring to an embodiment using a magnetite particle as a ferromagnetic iron oxide particle.

### <Synthesis of magnetite particle>

The magnetite particle can be synthesized by the following method comprising oxidation of iron salt in an aqueous solution. First, to an aqueous solution of divalent Fe ion, in which ferrous sulfate (FeSO₄·6H₂ O) has been dissolved, is added dropwise an NaOH aqueous solution to allow precipitation of ferrous hydroxide [Fe(OH)₂]. This ferrous hydroxide suspension is adjusted to pH 9-10 and oxidized by blowing air to grow a magnetite particle.

When the pH is smaller than the above-mentioned range, precipitation of magnetite becomes slow. When the pH is greater than the above-mentioned range, goethite (α-FeOOH) tends to grow. The flow rate of the air and retention temperature of the suspension greatly affect the particle size of the magnetite particle. The flow rate of the air is desirably controlled to 100-400 liter/hour and the retention temperature of the suspension is desirably controlled to 50-90°C. Generally, a higher flow rate of the air accelerates the crystal growth of the magnetite and the particle size becomes smaller. When the flow rate of the air is too small or too large, substances other than magnetite are easily mixed during precipitation. A higher retention temperature causes easy crystal growth of the magnetite and the particle size becomes large. When the retention temperature is too low, goethite (α-FeOOH) particles grows easily.

According to the method above, a magnetite particle having an average particle size of 0.05-0.5 µm, preferably 0.1-0.5 µm, can be synthesized. The "particle size" of the magnetite particle (ferromagnetic iron oxide particle) means that of the aforementioned magnetic carrier, and the average particle size of the magnetite particle (ferromagnetic iron oxide particle) can be calculated in the same manner as in the average particle size of the aforementioned magnetic carrier. In other words, the average particle size can be determined by measuring the size of 300 particles on a scanning electron microscope (SEM) image and calculating the average value.

### <Adhesion of silica>

As a ferromagnetic iron oxide particle, a separately obtained dry particle may be dispersed in water. However, it is preferable to apply a ferromagnetic iron oxide particle obtained as mentioned above to coating with silica, without going through a drying step. This is because use in the form of a suspension containing water results in good dispersibility, shows fine wettability to silica and permits uniform coating of ferromagnetic iron oxide particle with silica.

For coating of silica, the following two methods can be employed.

### (First method of silica coating)

A magnetite particle is washed thoroughly with pure water to give a suspension containing water, without drying. To the suspension is added a predetermined amount of sodium silicate (water glass), which is dissolved therein. The amount of sodium silicate to be added is preferably 10-300 wt%, more preferably 15-250 wt%, of the magnetite particle, upon conversion to silica (SiO₂). When it is less than 10 wt% relative to the magnetite particle, higher saturation magnetization can be easily obtained but uniform coating of magnetite particle with silica tends to be difficult. When it exceeds 300 wt% relative to the magnetite particle, the saturation magnetization becomes too small, and may degrade the responsiveness to the magnetic field when prepared into a magnetic carrier.

A surfactant solution wherein a surfactant is dissolved in an organic solvent is separately prepared. This surfactant solution is mixed with a magnetite particle suspension, in which the above-mentioned sodium silicate has been dissolved.

While the surfactant is free of any particular limitation, a sorbitan fatty acid ester surfactant is preferable because it has both the hydrophilicity and the hydrophobicity. Sorbitan fatty acid ester surfactant is exemplified by sorbitan monostearate, sorbitan monolaurate, sorbitan monopalmitate, sorbitan monooleate, sorbitan trioleate and the like, of which sorbitan monooleate is particularly preferable, because the balance of the hydrophilicity and hydrophobicity is suitable for achieving the object of the present invention.

The organic solvent preferably has low solubility in water to achieve an emulsion state, such as benzene, toluene, xylene, n-hexane, isohexane, cyclohexane, ethyl acetate, butyl acetate and the like. Of these, n-hexane is particularly preferable because it shows superior operability after silica coating, in extracting a magnetic carrier from the reaction mixture, washing thereof and the like.

A mixture of the above-mentioned surfactant solution is stirred using a mixer such as homomixer, homogenizer and the like to prepare a W/O type suspension containing emulsion particles. The emulsion particle has a structure wherein a magnetite particle and an aqueous sodium silicate solution are included in a surfactant in an organic solvent.

While the mixing time varies depending on the mixer to be used, it is preferably about 1-30 min. When the mixing time is too short, uniformly sized emulsion particles are difficult to obtain. When the mixing time is too long, a magnetite particle (ferromagnetic iron oxide particle) and silica react due to mixing energy, which often results in the production of emulsion particle without the aforementioned structure.

The suspension containing the emulsion particle thus obtained is added dropwise to an aqueous ammonium salt solution. By the dropwise addition, sodium silicate is neutralized by ammonium salt and precipitates as silica. Sodium silicate dissolves in alkaline water but is insoluble in neutral water. As a result, a spherical particle of magnetite particle coated with silica is produced. As the above-mentioned ammonium salt, sulfate and carbonate are preferably used, and ammonium carbonate, ammonium bicarbonate, ammonium sulfate and the like are preferable.

The dropwise addition of the suspension into an aqueous ammonium salt solution is preferably performed to allow gradual precipitation of silica. The time of dropwise addition is preferably 10 min-3 hrs. When the time of dropwise addition is less than 10 min., the obtained silica layer tends to have a defect (e.g., big clearance in silica layer), and magnetic carrier concaves and convexes on the surface. Even when the time of dropwise addition exceeds 3 hrs., it poses no particular problem in terms of properties but a long synthesis time is meaningless.

The particle thus synthesized is washed thoroughly with pure water, filtrated and dried in air. The drying temperature is preferably 40-120°C, more preferably 60-100°C. When the drying temperature is less than 40°C, water taken into the magnetic carrier cannot be sufficiently removed easily. When the drying temperature exceeds 120°C, saturation magnetization tends to become lower due to oxidation of ferromagnetic iron oxide particle. For sufficient drying without effect on the property of the magnetic carrier, the drying time is preferably 1-24 hrs., more preferably 2-20 hrs.

In this way, ferromagnetic iron oxide particle is coated with silica, and a particle having an average particle size of preferably 0.1-10 µm can be obtained. The thus-obtained particle is subjected to the heat treatment to be mentioned later to give a magnetic carrier capable of meeting the above-mentioned [1] - [3].

### (Second method of silica coating)

In the second method, too, the magnetite particle (ferromagnetic iron oxide particle) as prepared as above is sufficiently washed with water, and, without drying, suspended in water to give a suspension. In this case, the mixing ratio of magnetite particle and water is controlled to make the magnetite particle content 1-10 wt% of water. The magnetite particle content relative to water affects uniform adhesion of silica to the vicinity of the surface of the magnetite particle, and silica is most uniformly adhered when the magnetite particle content is within the above-mentioned range. When the magnetite particle content relative to water is less than 1 wt%, the concentration is too low and silica is easily precipitated in the part other than the surface of the magnetite particle. When the magnetite particle content relative to water exceeds 10 wt%, the concentration becomes too high and the magnetite particle easily flocculates, making uniform adhesion of silica near the surface of each magnetite particle difficult.

Then, sodium silicate (water glass) is added to the above-mentioned suspension in a proportion of 3-100 wt%, preferably 3-50 wt%, relative to the magnetite particle upon conversion to silica (SiO₂). When the amount of sodium silicate to be added is less than 3 wt%, the amount of silica to be adhered to the surface of the magnetite particle becomes insufficient, making binding with biological substance difficult. When the amount of sodium silicate to be added exceeds 100 wt%, uniform adhesion of silica near the surface of each magnetite becomes difficult, which in turn reduces the effect of increased binding amount. In addition, because the amount of saturation magnetization as a magnetic carrier decreases, the collectability by the magnetic field tends to be degraded.

The amount of the above-mentioned sodium silicate to be added is preferably 0.3-2 wt%, more preferably 0.5-2 wt.%, relative to water upon conversion to silica (SiO₂). Preferably, the amounts of the magnetite particle, sodium silicate and water are controlled so that the magnetite particle content relative to water will be 1-10 wt%. As in the aforementioned first method, the ferromagnetic iron oxide particle is coated with silica by way of precipitation of silica by neutralization in the second method. During the silica precipitation, the liquid viscosity becomes high. When the viscosity becomes too high, uniform adhesion of silica to the vicinity of the surface of each magnetite particle becomes difficult. In contrast, when the above-mentioned viscosity is too low, precipitation of silica becomes difficult.

The particle thus synthesized is washed thoroughly with pure water, filtrated and dried in air. The drying temperature is preferably 40-120°C, more preferably 60-100°C. When the drying temperature is less than 40°C, water taken into the magnetic carrier cannot be sufficiently removed easily. When the drying temperature exceeds 120°C, saturation magnetization tends to become lower due to oxidation of ferromagnetic iron oxide particle. For sufficient drying without effect on the property of the magnetic carrier, the drying time is preferably 1-24 hrs., more preferably 2-20 hrs.

When compared to the aforementioned first method, this second method is suitable for the production of a magnetic carrier having a smaller average particle size, particularly an average particle size of not more than 1 µm. By applying the particle thus obtained to the heat treatment to be mentioned below, a magnetic carrier capable of the above-mentioned [1]-[3] can be obtained.

### <Heat treatment>

The magnetic carrier thus synthesized shows superior performance as a magnetic carrier for extraction and purification of nucleic acid or purification of nucleic acid amplification product. When this magnetic carrier is heat treated, the property is further improved. The temperature of heat treatment is preferably 200-800°C, more preferably 250-500°C. When the temperature of heat treatment is less than 200°C, the effect of the heat treatment to be mentioned below is difficult to obtain. When the temperature exceeds 800°C, sintering occurs between the silica layers coating the ferromagnetic iron oxide particles, and magnetic carrier is frequently obtained in the form of an aggregation, which in turn degrades dispersibility of magnetic carrier in a solution, making isolation of nucleic acid difficult. The treatment time varies depending on the treatment temperature, but it is preferably 1-10- hrs. When the treatment time is too short, a sufficient effect of heat treatment is not obtained. When it is too long, magnetite particles easily aggregate.

While the reason for the preference for a heat treatment in the above-mentioned temperature range is not clear, the present inventors consider as follows. Magnetic properties such as saturation magnetization, coercive force and the like of the ferromagnetic iron oxide particles vary greatly depending on the heat treatment conditions after silica coating treatment of ferromagnetic iron oxide particles. This is considered to be attributable to the fact that magnetic carriers obtained by subjecting ferromagnetic iron oxide particles after coating with silica to heat treatments under various conditions show varying crystallinity and crystallite size of ferromagnetic iron oxide particle, even though the apparent particle size is the same, and the magnetic property of the ferromagnetic iron oxide particle greatly depends on the crystallinity and crystallite size. In general, when the crystallinity of a ferromagnetic iron oxide particle is improved, both saturation magnetization and coercive force tend to increase. When the crystal of ferromagnetic iron oxide particle grows (greater crystallite size), saturation magnetization increases but coercive force tends to decrease.

It is also possible that, due to the above-mentioned heat treatment, silica more firmly binds near the surface of ferromagnetic iron oxide particle and the crystallinity of silica is improved, as a result of which the bindability with nucleic acid is enhanced.

As used herein, the crystallinity of ferromagnetic iron oxide particle in the magnetic carrier refers to the completeness of the crystal structure a particle has, which is difficult to achieve quantitatively. For example, however, it can be determined qualitatively from the width of the diffraction peak in the X-ray diffraction method. When the crystallinity of particle becomes fine, the peak width becomes narrow. The ferromagnetic iron oxide of a particle magnetic carrier obtained by the above-mentioned heat treatment shows fine crystallinity as compared to the ferromagnetic iron oxide particle of conventional magnetic carriers obtained without the above-mentioned heat treatment. This can be examined from the above-mentioned width of the diffraction peak. That is, the ferromagnetic iron oxide particle of the magnetic carrier obtained by the above mentioned heat treatment has narrower peak width and finer crystallinity than those of the ferromagnetic iron oxide particles obtained without the heat treatment.

The crystallite size of a ferromagnetic iron oxide particle of a magnetic carrier means the size of each crystal constituting one ferromagnetic iron oxide particle. The crystallite size can be determined by the Scherrer Equation using the half value of diffraction peak by the X-ray diffraction method. The ferromagnetic iron oxide particle of a magnetic carrier obtained by the heat treatment under the above-mentioned conditions has a crystallite size, as determined by the above-mentioned X-ray diffraction method, of about 1.1-1.5 times that of conventional magnetic carriers obtained without the above-mentioned heat treatment and is markedly different.

The time of the above-mentioned heat treatment is not particularly limited and can be appropriately selected according to the temperature conditions of the above-mentioned heat treatment (generally, higher heat treatment temperature and longer heat treatment time result in improved crystallinity and crystal growth), but it is preferably 0.5-10 hrs., more preferably 1-5 hrs. A heat treatment of less than 0.5 hr. may not be able to afford sufficient effect of the aforementioned heat treatment. A heat treatment exceeding 10 hrs. easily causes sintering between silica layers covering the' ferromagnetic iron oxide particles and the magnetic carried often becomes an aggregation.

The above-mentioned heat treatment is preferably applied in an inert gas or reducing gas atmosphere. When the above-mentioned heat treatment is applied in an oxidizing gas atmosphere such as air or oxygen gas and the like, the effect can be afforded to some degree. However, when, for example, the ferromagnetic iron oxide particle is a magnetite particle, the magnetite may be oxidized by oxygen that invades through a fine defect and the like in silica, covering the ferromagnetic iron oxide particle, which may provide a maghemite (γ-Fe₂O₃) having still lower saturation magnetization, or excessively oxidized and non-magnetic hematite (α-Fe₂O₃). By applying the above-mentioned heat treatment in an inert gas or reducing gas atmosphere, inconveniences resulting from a heat treatment in an oxidizing gas atmosphere can be certainly obviated.

As the above-mentioned inert gas, nitrogen gas and argon gas are preferable. Particularly, nitrogen gas is preferably used because it is economic and convenient in handling.

As the above-mentioned reducing gas, hydrogen gas, carbon monoxide gas, town gas and the like can be mentioned. When the above-mentioned heat treatment is applied in a reductive atmosphere, a heat treatment in the presence of water vapor is preferable to prevent excessive reduction up to the state of a metal.

For prevention of undesirable oxidation of iron oxide particles, the above-mentioned heat treatment may be applied in a vacuum atmosphere.

In another preferable type (a second type) , a magnetic carrier is proposed where a ferromagnetic iron oxide particle is used as a magnetic particle, using this as a core substance, a compound comprising silicon and aluminum is adhered to the surface thereof. The magnetic carrier of the second type shows improved adhesion uniformity to a core substance, is superior in isolation and purification of nucleic acid, and provides a magnetic carrier for nucleic acid, which is superior in surface smoothness and flowability. The second type is not covered by the claims.

In a micro TAS, the magnetic carrier to retain nucleic acid is required to have a smallest possible particle size and superior flowability to flow in the system according to the magnetic field. The flowability is influenced by the surface smoothness of the magnetic carrier. Because flowability of the magnetic carrier can be improved as a compound for nucleic acid binding, such as silica and the like, is more uniformly adhered to the core substance, it is important that a coating of the above-mentioned compound such as silica and the like should be formed to uniformly cover the surface of each core substance particle.

From the above-mentioned aspects, the present inventors have used a ferromagnetic iron oxide particle as a core substance and considered from various aspects as regards the compound for.nucleic acid binding, which adheres to the surface of the ferromagnetic iron oxide particle. As a result, they have found that a compound containing aluminum together with silicon can uniformly adhere to the surface of the core substance particle. In particular, when this compound is a mixed oxide of silicon and aluminum, the effect thereof becomes remarkable. Since a compound comprising silicon and aluminum tends to have a compact structure, uniform adhesion to the surface of the core substance particle is considered to be improved.

In a compound comprising silicon and aluminum, the aluminum content is preferably 0.1-40 wt%, more preferably 0.2-30 wt%, of the total amount of the silicon and aluminum, When the aluminum content is less than 0.1 wt%, uniform adhesion to a ferromagnetic iron oxide particle is degraded and when it exceeds 40 wt%, the nucleic acid binding efficiency tends to be degraded. The aluminum content was measured using an X-ray Fluorescence Spectrometer JSX-3220ZS manufactured by JEOL Ltd. Mixtures of silica and alumina with various mixing ratios were measured to plot calibration, curves in advance, based on which the aluminum content was determined.

While the method of adding aluminum is not particularly limited, a mixed oxide consisting of silicon and aluminum is preferable, such as an oxide represented by a SiO₂ - Al₂O₃ composition. A compound comprising silicon and aluminum can be present as a mixture with a magnetic particle, but preferably forms a coating on the surface of a magnetic particle.

In addition, a compound comprising silicon and aluminum content is preferably 3-100 wt%, particularly preferably 5-80 wt%, relative to the ferromagnetic iron oxide particle. When the content of the above-mentioned compound is less than 3 wt%, nucleic acid binding becomes insufficient. When the content exceeds 100 wt%, the compound easily precipitates in the part other than the particle surface. Particularly when the above-mentioned compound is form on a surface of a ferromagnetic iron oxide particle, the binding efficiency tends to be degraded due to the occurrence of flocculation and the like of the particle.

The ferromagnetic iron oxide particle, on which a compound comprising silicon and aluminum is to be coated, may be similar to that of the claims type. While the particle size of the ferromagnetic iron oxide particle is not particularly limits, but the range of 0.05-0.5 µm is most suitable in view of the dispersibility in an aqueous solution.

In the second type, the saturation magnetization, coercive force and average particle size of the magnetic carrier after forming a compound comprising silicon and aluminum are not particularly limited, but it is preferably within the aforementioned range of [1] - [3].

The magnetic carrier bound with a nucleic acid via a compound comprising silicon and aluminum is collected by a magnet and the like. The collectability depends on tha amount of the saturation magnetization of the magnetic carrier, wherein a greater amount of saturation magnetization results in higher improvement of collectability. When the content of the above-mentioned compound is 3-100 wt% of the ferromagnetic iron oxide particle, a decrease in the amount of saturation magnetization does not substantially influence the collectability with a magnet. When the content of the above-mentioned compound is less than 3 wt%, the bindability with nucleic acid becomes insufficient, and when it exceeds 100 wt%, the saturation magnetization of the magnetic carrier becomes small; which tends to degrade collectability afforded by a magnet.

The magnetic carrier collected in this way by a magnet is transferred to a different solution and allows elution of the nucleic acid bound with the above-mentioned compound into this solution. The magnetic carrier of the present invention has been found to show superior elution property.

In addition, the magnetic carrier of the second type preferably has a spherical or granular particle shape to afford most superior dispersibility, as in the magnetic carrier of the claims.

The ferromagnetic iron oxide particle preferably used for the magnetic carrier of the second type is similar to those preferably used for the magnetic carrier of the type claims. Particularly, those obtained using the magnetite particle, adhering a compound comprising silicon and aluminum to the vicinity of the surface of the particle and heat treatment to convert the compound comprising silicon and aluminum to an oxide are superior in flowability and particularly suitable as a magnetic carrier to be used for micro TAS.

In the following, a production method of the magnetic carrier of the second type is explained by referring to an embodiment using a magnetite particle as a ferromagnetic iron oxide particle. The synthesis of the magnetite particle is the same as in the type claims.

### <Adhesion of compound comprising silicon and aluminum>

A method using the above-mentioned magnetite particle as a ferromagnetic iron oxide particle, which comprises adhering a compound comprising silicon and aluminum to the surface thereof is explained. First, as in the (second method of silica coating) under the aforementioned <Adhesion of silica>, a suspension of magnetite particles is prepared. A preferable amount of magnetite particle relative to water is 1-10 wt%.

Separately from the suspension of magnetite particle, an aluminum salt is dissolved in an aqueous alkaline solution. The amount of the aluminum salt to be added upon conversion to aluminum is preferably 0.1-40 wt% relative to silicon to be mentioned below, in view of uniform adhesion. While aluminum salt is not particularly limited, aluminum chloride, aluminum acetate, aluminum nitrate, aluminum sulfate and the like are preferably used. As the aqueous alkaline solution, aqueous sodium hydroxide solution, aqueous potassium hydroxide solution, aqueous lithium hydroxide solution and the like are used.

The aqueous aluminum salt solution preferably has a pH of 11-14. When the pH is less than 11, subsequent mixing of this solution with silicate may lead to precipitation of a silicon and/or aluminum compound.

As the silicate, sodium silicate is generally used. The aqueous sodium silicate solution (water glass) is added to the above-mentioned aqueous solution of aluminum salt to give a mixed solution of silicate and aluminum salt. The amount of the silicate is such an amount as makes the amount of a compound comprising silicon and aluminum 3-100 wt% relative to the magnetite particle. When it is less than 3 wt%, the amount of the above-mentioned compound to be adhered near the surface of the magnetite particle becomes insufficient, the amount of nucleic acid binding becomes small, and extraction efficiency is degraded. When it exceeds 100 wt%, the above-mentioned compound cannot be uniformly adhered near the surface of each magnetite particle. In addition, the amount of saturation magnetization of the magnetic carrier decreases and collectability by the magnetic field is degraded.

A mixed solution of silicate and aluminum salt thus adjusted alkaline and a suspension of the aforementioned magnetite particle are mixed, the above-mentioned particles are dispersed in the above-mentioned mixed solution, and an acid (generally acidic aqueous solution of hydrochloric acid, phosphoric acid and the like) is added to neutralize to the pH range of 7-8 to allow precipitation of a compound comprising silicon and aluminum. A magnetic carrier can be synthesized in this manner by adhering the compound comprising silicon and aluminum near the surface of the magnetite particle, washing thoroughly, filtrating and drying in air at a given temperature for a given time.

### <Heat treatment>

The thus-synthesized magnetic carrier of the second type can be used as a magnetic carrier for extraction and purification of nucleic acid or purification of nucleic acid amplification product. Application of heat treatment to this magnetic carrier further improves its properties.

The heat treatment is preferably performed in an inert gas such as nitrogen, argon and the like. The heat treatment may be performed in vacuum. While an oxidizing gas such as air can be used, a high heating temperature causes oxidation of magnetite particle to easily lower the saturation magnetization. Accordingly, use of an inert gas is preferable.

The heat treatment temperature is preferably 100-800°C. when it is lower than 100°C, the effect of heat treatment is small. When it exceeds 800°C, magnetite particles are easily sintered by heating, thereby degrading dispersibility during binding and eluting nucleic acid. While the heat treatment time varies depending on the heat treatment temperature, it is generally preferably 1-10 hrs. When the heat treatment time is short, a sufficient effect of heat treatment cannot be obtained, and when it is too long, the magnetite particles are easily sintered.

By such heat treatment, a compound comprising silicon and aluminum, particularly a mixed oxide of the above-mentioned both elements, more firmly binds near the surface of the magnetite particle, whereby magnetic carrier (the present invention, the second embodiment) superior in bindability with nucleic acid, and simultaneous collectability by magnetic field and dispersibility in removal of magnetic field/elution property of nucleic acid can be obtained.

The magnetic carrier obtained by the above-mentioned method, is superior in adhesion uniformity of a compound comprising silicon and aluminum to a core particle. The adhesion uniformity can be also evaluated by the volume of sedimentation when a magnetic carrier is dispersed in water.

That is, the smaller the sedimentation volume is, the smaller the flocculation of magnetic carrier, which demonstrates that the compound comprising silicon and aluminum uniformly adheres to a magnetite particle. This is based on the fact that when magnetic carriers, wherein the above-mentioned compound is uniformly adhered to the surface of the magnetic particles, are dispersed in water and stood, still, they become virtually closest-packed. In contrast, in magnetic carriers wherein the above-mentioned compound is non-uniformly adhered to the surface of the magnetic particle, a compound comprising silicon and aluminum forms a steric hindrance which prevents easy closest packing. As a result, the sedimentation volume becomes large.

An embodiment of the magnetic carrier of the present invention is organized once again into the following (1) - (6).
(1) A magnetic carrier wherein silica in an amount of 3-100 wt% of the ferromagnetic iron oxide particle is adhered near the surface of each ferromagnetic iron oxide particle,
(2) this ferromagnetic iron oxide particle is a magnetite particle,
(3) this magnetic carrier has a spherical or granular shape and has an average particle size of 0.12-0.45 µm.
(4) the coercive force and saturation magnetization of the magnetic carrier are 2.39-11.94 kA/m (30-150 oersted) and 30-80 A·m²/kg (30-80 emu/g), respectively,
(5) in a preferable production method, silica is adhered in an aqueous suspension of this ferromagnetic iron oxide particle by adjusting the amount of sodium silicate on conversion to SiO₂ to 0.3-2 wt% of water, and the amount of ferromagnetic iron oxide particle to 1-10 wt% relative to water,
(6) after adhesion of silica, the carrier is washed with water, dried and preferably subjected to heat treatment in an inert gas.

Another type of magnetic carrier, athough not claimed, is organized once again into the following (1)' - (5)'.
(1)' A magnetic carrier comprising a ferromagnetic iron oxide particle and silica coating said particle.
(2)' the ferromagnetic iron oxide particle is any of magnetite, maghemite or an intermediate iron oxide particle, particularly preferably a magnetite particle,
(3)' this ferromagnetic iron oxide particle is synthesized by oxidation in an aqueous solution of iron salt, and without going through a drying step, subjected to a silica coating treatment in a suspension containing water,
(4)' after .silica coating, the carrier is washed with water, dried and subjected to heat treatment in an inert gas or reducing gas atmosphere at 200-800°C, and
(5)' has a saturation magnetization of 30-80 A-m²/kg (30-80 emu/g), a coercive force of 2.39-11.94 kA/m (30-150 oersted) and an average particle size of 0.1-10 µm.

Another type of magnetic carrier, athough not claimed, is organized once again into the following (1)''- (8)''.
(1)" A magnetic carrier wherein a compound comprising silicon and aluminum in an amount of 3-100 wt% of the ferromagnetic iron oxide particle is adhered near the surface of each ferromagnetic iron oxide particle,
(2) " the above-mentioned ferromagnetic iron oxide particle is a magnetite particle,
(3)" the above-mentioned compound is an oxide, .
(4)" the aluminum content of the above-mentioned compound is preferably 0. 1-40 wt% of the total amount of silicon and aluminum,
(5)" this magnetic carrier has a spherical or granular shape and an average particle size of 0.1-10 µm,
(6)'' the coercive force and saturation magnetization of the magnetic carrier are preferably 0.80-15.92 KA/m (10-200 oersted) and 10-80 A·m²/kg (10-80 emu/g), respectively,
(7)" the production method preferably comprises dispersing a ferromagnetic iron oxide particle in a mixed solution of silicate and aluminum salt, and adding an acid for neutralization to precipitate a compound comprising silicon and aluminum,
(8)" after the above-mentioned precipitation, the carrier is preferably washed with water, filtrated, dried and subjected to heat treatment in an inert gas.

In the present specification, by the "magnetic carrier for biological substance" is meant a magnetic particle (powder) used for binding a biological substance by bringing the carrier into contact with the biological substance in an aqueous solution of a sample containing said biological substance. Therefore, the present invention encompasses the use of the aforementioned magnetic particle (powder) for binding a biological substance by bringing the carrier into contact with the biological substance in an aqueous solution of a sample containing said biological substance. Specific operation methods and the like relating to the use are mentioned below, it being understood that said methods of the invention are defined by the claims.

The isolation method of the present invention is characterized by the steps of [i] bringing a biological substance into contact with the aforementioned particular magnetic carrier in an aqueous solution of a sample containing a biological substance to form a complex wherein the biological substance and the magnetic carrier are bound, [ii] separating the above-mentioned complex from the sample by an external magnetic field, and [iii] eluting the biological substance from a complex separated from the sample, whereby the biological substance is isolated from the sample.

The "biological substance" as used in the present invention refers to, for example, a substance contained in a sample derived from a living organism, such as bacteria, yeast, Fungus, insect cell, zooblast, animal tissue, plant tissue, archaebacteria and the like. For example, nucleic acid such as DNA and RNA, various proteins such as enzyme, antibody and the like, and various polysaccharides can be used. Of the above-mentioned examples, nucleic acid binds with silica in the presence of high concentration chaotropic ion. Therefore, nucleic acid is particularly preferable as a biological substance to be isolated from the sample by the isolation method of the present invention.

In the following, the method of the present invention is described in detail referring to nucleic acid as a biological substance.

In the above-mentioned step [i], an aqueous solution containing a sample containing the objective nucleic acid is mixed with the aforementioned particular magnetic carrier to bring the nucleic acid into contact with a magnetic carrier. As used herein, an aqueous solution containing a sample containing nucleic acid includes a liquid wherein a sample is not completely dissolved in water. The method for binding the nucleic acid with the magnetic carrier is free of any particular limitation as long as they are mixed in a suitable buffer to bring them into contact with each other. The mixing is sufficiently achieved by, for example, gently reversing the tube to allow stirring or shaking the tube, which is done using, for example, a commercially available vortex mixer and the like. In step [i], a complex binding nucleic acid with magnetic carrier is formed.

In the above-mentioned step [i], a solution for nucleic acid extraction, in which a magnetic carrier is dispersed in a buffer containing a chaotropic substance, EDTA (ethylenediamine tetraacetic acid), trishydrochloric acid and the like, is preferably prepared in advance: Such solution for nucleic acid extraction is preferably prepared to achieve the concentration of the magnetic carrier of 0.02-0.5 g/mL, more preferably 0.2-0.45 g/mL. When the concentration of the above-mentioned magnetic carrier is less than 0.02 g/mL, retention of many nucleic acids becomes difficult and magnetic collectivity tends to become poor. When the concentration of the above-mentioned magnetic carrier exceeds 0.5 g/mL, dispersibility and preservation stability of the solution for nucleic acid extraction also tends to become poor. As the chaotropic substance to be contained in the solution for nucleic acid extraction, at least one of guanidine salt, sodium iodide, potassium iodide, sodium (iso)thiocyanate, urea and the like is used. The concentration of chaotropic substance in a solution for nucleic acid extraction is preferably 1-10 mol/L.

The mixing ratio of the magnetic carrier in an aqueous solution containing sample is preferably such a ratio as makes volume ratio of the magnetic carrier and the aqueous solution containing a sample (magnetic carrier/aqueous solution) 1/100-1/10.

In the subsequent step [ii], the nucleic acid bound with the magnetic carrier in the above-mentioned step [i] is separated from the sample as a complex of a magnetic carrier and a nucleic acid. For separation, an external magnetic field is utilized optionally together with an external electric field. Specifically, a method comprising separation of solution can be mentioned, wherein a magnet is placed near the side wall of a tube containing an aqueous solution containing a magnetic carrier bound with a biological substance to collect the magnetic carrier bound with a biological substance to the side wall.

As a magnet to be used as an external magnetic field, a magnet having magnetic induction of 2000-3000 gauss is preferably used. When an external magnetic field is applied with magnetic induction of less than 2000 gauss, the sensitivity to the magnetic field tends to become weak, resulting in degraded collection performance of magnetic carrier, and when an external magnetic field is applied with magnetic induction exceeding 3000 gauss, an apparatus to produce magnetic field becomes bulky and costly

In step [iii], the nucleic acid is eluted from a complex of a magnetic carrier and a nucleic acid separated from the above-mentioned sample. In this step, for example, a chaotropic substance is washed several times with a washing solution capable of washing the substance, the magnetic carrier is dried, after which a solution for elution capable of dissolving the nucleic acid, such as a solution having a low ion concentration such as TE buffer and the like or sterile water is injected to cause elution of the nucleic acid from the magnetic carrier into a solution for elution, whereby a nucleic acid can be finally recovered.

The above-mentioned washing solution is not particularly limited as long as a chaotropic substance can be dissolved, and conventionally employed acetone, alcohol diluted with water and the like are exemplarily shown. From the aspects of cost and safety, use of 70% alcohol is preferable. As the above-mentioned solution for elution, the liquid capable of dissolving the nucleic acid is not particularly limited and exemplified by sterile water, TE buffer, trishydrochloric acid buffer and the like. For convenience, use of sterile water and TE buffer (20 mM trishydrochloric acid, 1 mM EDTA, pH 7.5) is preferable.

As mentioned above, the magnetic carrier preferably used in the present invention (a) can disperse in an amount of at least 20 mg in 1 mL of an aqueous solution of a sample containing a biological substance, (b) can be collected by not less than 90 wt% within 3 seconds in the presence of a magnetic field of 2000-3000 gauss, and (c) can reversibly bind with at least 0.4 µg of a biological substance per 1 mg thereof, showing strikingly superior ability as compared with conventional carriers.

By the use of such magnetic carrier, due to the expression of the ability of the above-mentioned (a), the magnetic carrier can be efficiently contacted with a biological substance in an aqueous solution of a sample in the above-mentioned step [i], whereby a complex, in which a magnetic carrier and a biological substance are bound, can be efficiently formed. This effect can be synergistically enhanced by the exertion of the ability of the above-mentioned (c), whereby the efficiency of complex formation can be enhanced. In addition, due to the expression of the ability of the above-mentioned (b), the complex can be certainly and rapidly collected by the external magnetic field in the above-mentioned step [ii] . Furthermore, due to the expression of the ability of the above-mentioned (c), the biological substance can be efficiently eluted in the above-mentioned step [iii] from the complex separated from the sample.

In this way, by simultaneous achievement of the abilities of the above-mentioned (a) - (c) of the magnetic carrier, an isolation method of biological substances, which is superior in dispersibility in aqueous solutions as well as in the collectability due to the magnetic field, superior in reversible binding ability with a biological substance and elution property of the bound biological substance, as compared to conventional ones, and affording strikingly improved isolation and purification efficiency of a biological substance, as compared to conventional methods, can be realized.

While the aforementioned isolation method has been explained when the biological substance is a nucleic acid, biological substances that can be isolated from a sample by the isolation method of the present invention is not limited to a nucleic acid. When the objective biological substance is a protein or polysaccharide, for example, it can be bound with a magnetic carrier by forming an appropriate functional group (amino group, carboxyl group, phosphoric acid group and the like) on the surface of a magnetic carrier (outer surface of silica layer) using a silane coupling agent, as conventionally known. In step [iii], when the biological substance is a protein, a magnetic carrier bound with the protein is washed. several times with phosphate buffer, tris-HCl buffer and the like and eluted by addition of a buffer containing a protein ligand, and when the biological substance is a polysaccharide, it can be eluted by lowering the ionic strength of a solution for elution or heat treatment.

### Examples

The present invention is explained in detail in the following by referring to Examples, which are not to be construed as limitative.

### Example 1

### <Synthesis of magnetite particle>

Ferrous sulfate (FeSO₄·7H₂O, 100 g) was dissolved in pure water (1000 cc). Sodium hydroxide (28.8 g) was dissolved in' 500 cc of pure water to achieve equimolar with the ferrous sulfate. Then aqueous solution of sodium hydroxide was added dropwise over 1 hr. while stirring to an aqueous ferrous sulfate solution to precipitate ferrous hydroxide. After the completion of the dropwise addition, the suspension containing the precipitated ferrous hydroxide was heated to 85°C with stirring. After the temperature of the suspension reached 85°C, the reaction mixture was oxidized for 8 hrs. while blowing in air at a rate of 200 L/hr with an air pump to give magnetite particles. The magnetite particles were almost spherical and had an average particle size of 0.28 µm.

The average particle size of the magnetite particle was determined by measuring the size of 300 particles on a transmission electron microscopic photograph and calculating a number average thereof.

### <Adhesion of silica>

After thorough washing of a suspension of the above-mentioned magnetite particle with pure water, the weight of the magnetite particle and water was adjusted to 10 g and 200 g, respectively, without drying. The amount of the.magnetite in the suspension after water washing was determined by sampling and drying a part thereof. In this suspension was dissolved 3.6 g of sodium silicate.

While the above-mentioned sodium silicate is alkaline in a dissolution state, when adjusted to near neutral by a neutralization step, it precipitates as silica. Therefore, to this magnetite particle suspension, in which sodium silicate had dissolved, was dropwise added dilute hydrochloric acid with stirring over about 1 hr. and adjusted to near neutral. After the completion of the dropwise addition, the mixture was stirred for 1 hr. By this step, silica was adhered near the surface of the magnetite particle.

In this reaction, the amount of sodium silicate and the amount of magnetite particle, relative to water are important, and when the amount of sodium silicate on conversion to SiO₂ is 0.5-2 wt% relative to water, the liquid used to precipitate silica from an aqueous sodium silicate solution by neutralization has an optimal viscosity, whereby silica can be adhered uniformly near the surface of each magnetite particle. When the amount of magnetite particle relative to water is 1-10 wt%, silica can be preferentially adhered near the surface of the magnetite particle.

Then, stirring was stopped to allow natural sedimentation. The supernatant was removed, and after water washing, the resultant solid was filtrated and dried at 60°C for 4 hrs. to give a magnetic carrier for nucleic acid.

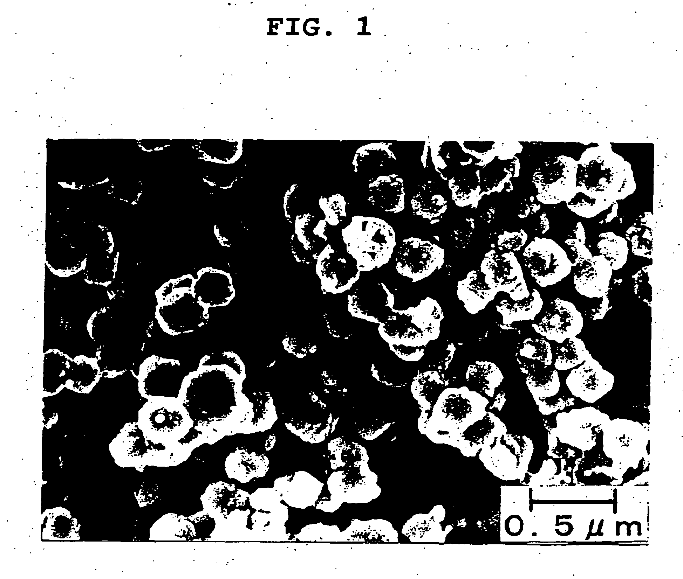

This magnetic carrier was spherical or granular and had an average particle size of 0.32 µm, a coercive force of 4.78 kA/m (60 oersted) and saturation magnetization of 66.8 A·m²/kg (66.8 emu/g). The amount of the coated silica was 19.4 wt% of magnetite particle on conversion to SiO₂. Fig. 1 shows an SEM image of the magnetic carrier. From this image, it is observed that silica adheres near the surface of the magnetite particle.

### Example 2

In the same manner as in Example 1 except that the amount of sodium silicate was changed from 3.6 g to 1.8 g in the silica application step, silica was applied to magnetite particles to give a magnetic carrier for nucleic acid.

This magnetic carrier was spherical or granular, and' showed an average particle size of 0.29 µm, coercive force of 4.38 kA/m (55 oersted) and a saturation magnetization of 75.1 A·m²/kg (75.1 emu/g). The amount of the coated silica was 9.8 wt% relative to the magnetite particle upon conversion to SiO₂. By SEM, adhesion of silica in the vicinity of the surface of each magnetite particle was observed.

### Example 3

In the same manner as in Example 1 except that, in the silica application step, the weight of the magnetite particle and that of water were changed from 10 g and 200 g to 10 g and 500 g, respectively, and the amount of sodium silicate was changed from 3.6 g to 14.9 g, silica was applied to magnetite particles to give a magnetic carrier for nucleic acid.

This magnetic carrier was spherical or granular, and showed an average particle size of 0.34 µm, coercive force of 5.97 kA/m (75 oersted) and a saturation magnetization of 60.1 A·m²/kg (60.1 emu/g). The amount of the coated silica was 78.9 wt% relative to the magnetite particle upon conversion to SiO₂. By SEM, adhesion of silica in the vicinity of the surface of each magnetite particle was observed.

### Example 4

The magnetic carrier obtained in Example 1 was subjected to a heat treatment in nitrogen gas at 500°C for 2 hours.

This magnetic carrier was spherical or granular, and showed an average particle size of 0.32 µm, a coercive force of 5.18 (65 oersted) and a saturation magnetization of 68.3 A·m²/ kg (67.3 emu/g). The.amount of the coated silica was 19.4 wt% relative to the magnetite particle upon conversion to SiO₂. By SEM, adhesion of silica in the vicinity of the surface of each magnetite particle was observed.

### Example 5

In the same manner as in Example 1 except that the temperature of a suspension containing precipitate of ferrous hydroxide was changed from 85°C to 60°C in the synthesis step of magnetite particle, a magnetite particle having an average particle size of 0.13 µm was synthesized.

Using this magnetite particle and in the same manner as in Example 1, silica was applied to give a magnetic carrier for nucleic acid.

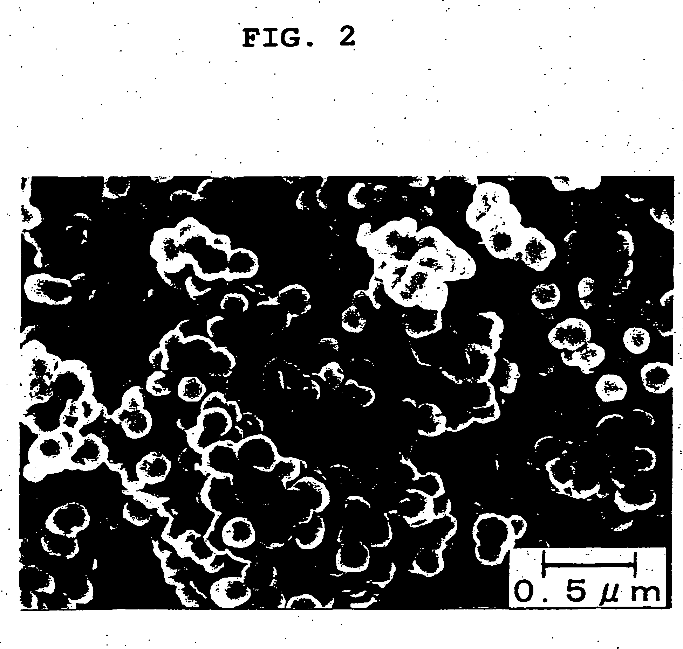

This magnetic carrier was spherical or granular, and showed an average particle size of 0.17 µm, a coercive force of 7.57 kA/m (95 oersted) and a saturation magnetization of 63.4 A·m²/kg (63.4 emu/g). The amount of the coated silica was 19.8 wt% relative to the magnetite particle upon conversion to SiO₂. Fig. 2 shows an SEM image of this magnetic carrier, wherein adhesion of silica in the vicinity of the surface of each magnetite particle can be observed.

### Comparative Example 1

Commercially available maghemite (γ-Fe₂O₃) particles were used as ferromagnetic iron oxide particles. The maghemite particles have an average particle size of about 0.26 µm, a coercive force of 8.76 kA/m (110 oersted) and a saturation magnetization of 83.5 A·m²/kg (83.5 emu/g).

Water (25 g) was added to suspend said maghemite particles (5 g). Sodium silicate (28 g) was added to this suspension and dissolved. Sorbitan monolaurate (1.44 g) was dissolved in hexane (96 g), and this solution was mixed with the above-mentioned sodium silicate dissolved maghemite suspension. The mixture was stirred by a homomixer to give an emulsion.

Ammonium sulfate (64 g) was dissolved in pure water (288 cc), and while stirring the solution, the above-mentioned emulsion was added dropwise thereto to give a magnetic carrier comprising a maghemite particle coated with silica, which was washed with water and dried at 60°C.

The magnetic carrier thus obtained showed an average particle size of about 5.6 µm, which was far larger than that of 0.1-0.5 µm of the magnetic carrier of the present invention. The coercive force was 7.33 kA/m (92 oersted) and saturation magnetization was 22.1 A·m²/kg (22.1 emu/g). The amount of the coated silica was 260 wt% relative to maghemite particles upon conversion to SiO₂. In this magnetic carrier, the silica coating includes flock of maghemite particles, unlike the magnetic carrier of the present invention wherein silica adheres near the surface of each magnetite particle, as confirmed from the SEM image.

The average particle size, coercive force, saturation magnetization and amount of silica coating on magnetite particles (maghemite particle in Comparative Example 1) on conversion to SiO₂, as major properties of respective magnetic carriers for binding with nucleic acid, which were obtained by the above-mentioned Examples 1-5 and Comparative Example 1, are collectively shown in the following Table 1.

**Table 1**

| | average particle size (µm) | coercive force (kA/m) | saturation magnetization (A·m²/kg) | amount of silica coating (wt%) |
|---|---|---|---|---|
| Example 1 | 0.32 | 4.78 | 66.8 | 19.4 |
| Example 2 | 0.29 | 4.38 | 75.1 | 9.8 |
| Example 3 | 0.34 | 5.97 | 60.1 | 78.9 |
| Example 4 | 0.32 | 5.18 | 63.8 | 19.4 |
| Example 5 | 0.20 | 7.57 | 63.4 | 19.8 |
| Comp. Example 1 | 5.6 | 7.33 | 22.1 | 260 |

Then, the respective magnetic carriers for binding with nucleic acid, which were obtained in the above-mentioned Examples 1-5 and Comparative Example 1 were subjected to the following extraction tests. Nucleic acids were recovered by extraction from biological samples and recovery performance was examined. The results are as shown in Table 2.
(A) Reagent for extraction test
   (i) A magnetic carrier for nucleic acid was dispersed in sterile water to 0.2 mg/ml to give a dispersion solution.
   (ii) As a biological sample for isolation of nucleic acid, bacterial cells obtained by culturing *Escherichia coli* [*Escherichia coli* JM109 (available from Toyobo Co., Ltd., TAKARA SHUZO CO.. LTD., Invitrogen Corporation and the like)] in 3 mL TB medium/test tube at 37°C for 20 hrs. were used.
   (iii) As a solution for nucleic acid extraction, buffer A which is a buffer containing a chaotropic substance [7M guanidine hydrochloride (Nacalai Tesque, Inc.), and 50 mM Tris-HCl (Sigma), pH 7.5] was used.
   (iv) As a washing solution, buffer A which is a buffer containing a chaotropic substance [7M guanidine hydrochloride (Nacalai Tesque, Inc.), 50 mM Tris-HCl (Sigma), pH 7.5] was used.
   (v) As an agent for removing a high concentration salt, a 70% ethanol solutions and an acetone solution were used.
   (vi) As a solution for elution to recover nucleic acid bound with the magnetic carrier, sterile water was used.
(B) Extraction test method
   (1) The bacterial cell turbidity (OD660) was measured and the bacterial cells (OD660; 1.0) were prepared by centrifugal separation in an Eppendorf tube for 1.5 cc. Then, a solution for nucleic acid extraction (1,000 µl) was injected and mixed.
   (2) Thereafter, a dispersion (20 µl) of the magnetic carrier for nucleic acid was added.
   (3) While mixing every about 2 min., the mixture was left standing at room temperature for 10 min.
   (4) The above-mentioned tube was set on a magnet stand having a shape fitting the 1.5 cc Eppendorf tube to collect the magnetic carrier toward the magnet side.
   (5) The solution was sucked with a filter chip and discharged.
   (6) The tube was removed from the magnet stand and a.washing solution (1 cc) containing guanidine hydrochloride was poured therein.
   (7) After thorough mixing with the magnetic carrier, the tube was again placed on the magnet stand, and the solution was discharged in the same manner as above.
   (8) The washing step was repeated.
   (9) The magnetic carrier bound with nucleic acid was washed with 1 cc of 70% ethanol in the same manner as above and high concentration guanidine hydrochloride was removed.
   (10) The residue was again washed with 1 cc of 70% ethanol and 1 cc of acetone.
   (11) The above-mentioned tube was set in a heatblock at about 56°C, left standing for about 10 min. and acetone in the tube and in the magnetic carrier was removed by complete evaporation.
   (12) Sterile water (100 µl) was added to the magnetic carrier bound with nucleic acid by the above-mentioned method, and the above-mentioned tube was set in a heatblock at about 56°C and the mixture was left standing for about 10 min. while mixing every 2 min.
   (13) The tube was set on a magnet stand, the solution was recovered by suction with a filter tip, and transferred to a fresh tube. Generally, recovered amount was about 70 µl. Preservation was done at -70°C.
   (14) The thus recovered nucleic acid was determined for absorbance (OD 260 nm) with an absorptiometer and nucleic acid concentration was determined. This was multiplied by recovery volume and taken as nucleic acid recovery amount.

Of the above-mentioned operations, (1) - (3) correspond to the step of forming a complex wherein a magnetic carrier and a biological substance are bound, (4) - (5) correspond to the step of separating a complex from a sample and (6) - (12) correspond to the step of eluting a biological substance from a complex.

**Table 2**

| | recovered amount of nucleic acid (ng) |
|---|---|
| Example 1 | 2030 |
| Example 2 | 1960 |
| Example 3 | 1990 |
| Example 4 | 2050 |
| Example 5 | 2010 |
| Comparative Example 1 | 1070 |

As is clear from the above-mentioned results, the magnetic carriers of Examples 1-5, obtained by adhering silica in an amount of 3-100 wt% of the magnetite particle near the surface of each spherical or granular magnetite particle, had an average particle-size of 0.1-0.5 µm, a coercive force and a saturation magnetization of 2.39-11.94 kA/m (30-150 oersted) and 30-80 A-m²/kg (30-60 emu/g), respectively, were superior in isolation efficiency of nucleic acid, as compared to the magnetic, carrier of Comparative Example 1 having a structure where a flock of maghemite particles is included in silica.

This is attributable to the fact that, in the magnetic carriers of Examples 1-5, since silica adheres near the surface of each magnetite particle, the amount of silica capable of effectively binding with nucleic acid increases and silica also prevents flocculation of respective magnetite particles, the bindability with nucleic acid/collectability with magnetic carrier by magnetic field and dispersibility of magnetic carrier/elution property of nucleic acid are compatible.

### Reference Example 1

### (1) Synthesis of magnetite particle

In the same manner as in Example 1, a magnetite particle (suspension) was obtained. The magnetite particle was nearly spherical and had an average particle size of about 0.28 µm.

### (2) Silica coating treatment

A magnetite particle suspension was thoroughly washed with pure water and pure water was added without drying to make the total weight of this suspension 468 g. In this dispersion was dissolved 70 g of sodium silicate. Separately from the sodium silicate dissolved magnetite particle dispersion, 1500 cc of hexane solution, wherein 22.5 g of sorbitan monolaurate as a surfactant had been dissolved, was added to the above-mentioned suspension to give a mixed solution. This mixed solution was stir-dispersed by a homomixer (TOKUSHU KIKA KOGYO CO., LTD.) for 10 min. to give a dispersion. Then, 1000 g of ammonium sulfate was dissolved in 4500 cc of pure water. The above mentioned dispersion was dropwise added to this ammonium sulfate solution over about 1 hr. with stirring. After the completion of the dropwise addition, the mixture was stirred for 2 more hours for neutralization. By the neutralization with ammonium sulfate, silica was precipitated to enclose magnetite particles to form a coating (silica layer).

### (3) Heat treatment

The magnetite particle coated with silica obtained above was thoroughly washed with pure water, filtrated and dried in air at 60°C for 8 hrs. As mentioned above, the magnetite particles show different magnetic properties depending on the crystallinity and crystallite size of the particles. To control magnetic property, a heat treatment was applied. For heat treatment, the particles were heated in a nitrogen gas stream using a tubular furnace at 600°C for 2 hrs. After the heat treatment, the particles were cooled to room temperature while flowing a nitrogen gas, taken out to give a magnetic carrier. In the same manner as in the above-mentioned magnetite particles, measurement was done to find the average particle size of the magnetic carrier to be 5 µm. The observed shape of the magnetic carrier was spherical. The obtained magnetic carrier was subjected to the measurement using a vibrating sample magnetometer (TOEI INDUSTRY CO., LTD) and applying a magnetic field of 796.5 kA/m (10 kilooersted). As a result, the saturation magnetization was 47.1 A·m²/kg (47.1 emu/g) and coercive force was 5.18 kA/m (65 oersted).

The magnetic carrier was added to the objective aqueous solution of a sample containing a biological substance and dispersed in a vortex mixer to confirm if uniform suspension is obtained. As a result, it was found that at least 20 mg was dispersed in 1 mL of an aqueous solution of a sample containing a biological substance. This suspension was placed on a magnet and the weight of the magnetic carrier collected by the magnet in a given time was measured. As a result, it was found that, in the above-mentioned dispersion state, not less than 90 wt% thereof was collect within 3 seconds by a 2000-3000 gauss magnetic force. Moreover, by confirmation by quantitative determination analysis of nucleic acid before and after binding and dispersion, using nucleic acid as a biological substance, reversible binding with at least 0.4 µg of a biological substance per 1 mg of the magnetic carrier was observed.

### Reference Example 2

In the same manner as in Reference Example 1 except that ferrous hydroxide precipitate suspension was heated to 60°C during magnetite particle synthesis, a magnetic carrier was prepared. The obtained magnetic carrier was spherical and had an average particle size of 4 µm (average particle size of magnetite particle 0.13 µm), saturation magnetization of 45.3 A·m²/kg (45.3 emu/g), and a coercive force of 6.53 kA/m (82 oersted), as measured in the same manner as in Reference Example 1.

In the same manner as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 3

In the same manner as in Reference Example 1 except that a suspension of ferrous hydroxide precipitate was heated to 98°C during magnetite particle synthesis, a magnetic carrier was prepared. The obtained magnetic carrier was spherical and had an average particle size of 7 µm (average particle size of magnetite particle 0.42 µm), saturation magnetization of 47.5 A·m²/kg (47.5 emu/g), and a coercive force of 3.66 kA/m (46 oersted), as measured in the same manner as in Reference Example 1.

In the same manner as in Reference Example 1, it was confirmed that this magnetic carrier (at least 20 mg) could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 4

In the same manner as in Reference Example 1 except that the amount of sodium silicate dissolved in the magnetite particle dispersion was 58 g during the silica coating treatment, a magnetic carrier was prepared. The obtained magnetic carrier was spherical and had an average particle size of 4 µm, saturation magnetization of 51.2 A·m²/kg (51.2 emu/g), and a coercive force of 5.42 kA/m (68 oersted), as measured in the same manner as in Reference Example 1.

In the same manner as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 5

In the same manner as Reference Example 1 except that the amount of sodium silicate dissolved in the magnetite particle dispersion was 90 g during the silica coating treatment, a Magnetic carrier was prepared. The obtained magnetic carrier was spherical and had an average particle size of 6 µm, saturation magnetization of 40.3 A·m²/kg (40.3 emu/g), and a coercive force of 4.86 kA/m (61 oersted), as measured in the same manner as in Reference Example 1.

In the same manner as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 6

In the same manner as in Reference Example 1 except that a heat treatment was applied in a nitrogen gas stream at 800°C for 2 hrs., a magnetic carrier was prepared. The obtained magnetic carrier was spherical and had an average particle size of 5 µm, saturation magnetization of 47.8 A-m²/kg (47.8 emu/g), and a coercive force of 2 63 kA/m (33 oersted), as measured in the same manner as in Reference Example 1.

In the same manner as in Reference Example 1, it was confirmed that at least 20 mg this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 7

In the same manner as in Reference Example 1 except that a heat treatment was applied in a nitrogen gas stream at 400°C for 2 hrs., a magnetic carrier was prepared. The obtained magnetic carrier was spherical and had an average particle size of 5 µm, saturation magnetization of 43.0 A-m²/kg (43. 0 emu/g), and a coercive force of 8.37 kA/m (105 oersted), as measured in the same manner as in Reference Example 1.

In same as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 8

In the same manner as in Reference Example 1 except that argon gas was used as an atmosphere gas during heat treatment, a magnetic carrier was prepared. The obtained magnetic carrier was spherical and had an average particle size of 5 µm, saturation magnetization of 47.9 A-m²/kg (47.9 emu/g), and a coercive force of 5.34 kA/m (67 oersted), as measured in the same manner as in Reference Example 1.

In the same manner as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 9

In the same manner as in Reference Example 1 except that a heat treatment was applied using hydrogen gas as an atmosphere gas at 300°C for 2 hrs., a magnetic carrier was prepared. The obtained magnetic carrier was spherical and had an average particle size of 5 µm, saturation magnetization of 50.3 A·m²/kg (50.3 emu/g), and a coercive force of 4.06 kA/m (51 oersted). as measured in the same manner as in Reference Example 1.

In the same manner as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 10

As the magnetite particle, commercially available dry product (TODA KOGYO CORP.) was used. Such magnetite particle had a saturation magnetization of 83.5 A·m²/kg (83.5 emu/g), a coercive force of 8.76 kA/m (110 oersted) and an average particle size of 0.26 µm. Pure water was added to the above-mentioned magnetite particle to the total weight of 468 g to give a magnetite particle suspension.

In the same manner as in Reference Example 1 except that 70 g of sodium silicate was added and an ultrasonic dispersion apparatus was used for dispersion and a sodium silicate solution in which magnetite particles were dispersed was prepared, a silica coating treatment and a heat treatment were applied to give a magnetic carrier. The obtained magnetic carrier was spherical and had an average particle size of 6 µm, saturation magnetization of 40.1 A·m²/kg (40.1 emu/g), and a coercive force of 9.56 kA/m (120 oersted), as measured in the same manner as in Reference Example 1.

In the same manner as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 11

In the same manner as in Reference Example 1, synthesis of magnetite particle and a silica coating treatment were performed. The obtained magnetite particle coated with silica was thoroughly washed with pure water, filtrated, dried in air at 60°C for 8 hrs. to give a magnetic carrier without applying subsequent heat treatment. The obtained magnetic carrier was spherical and had an average particle size of 5 µm, saturation magnetization of 38.8 A·m²/kg (38.8 emu/g), and a coercive force of 7.81 kA/m (98 oersted), as measured in the same manner as in Reference Example 1.

In the same manner as in Reference Example 1, it was confirmed that at least 20 mg of this magnetic carrier could disperse in 1 mL of an aqueous solution of a sample containing a biological substance, in the above-mentioned dispersion state, not less than 90 wt% thereof was collected within 3 seconds by a 2000-3000 gauss magnetic force, and it could reversibly bind with at least 0.4 µg of a biological substance per 1 mg of the magnetic carrier.

### Reference Example 12

In the same manner as in Reference Example 5, synthesis of magnetite particle and a silica coating treatment were performed. The obtained magnetite particle coated with silica was thoroughly washed with pure water, filtrated, dried in air at 60°C for 8 hrs. to give a magnetic carrier without applying subsequent heat treatment. The obtained magnetic carrier was spherical and had an average particle size of 6 µm, saturation magnetization of 31-8 A·m²/kg (31.8 emu/g), and a coercive force of 8.76 kA/m (110 oersted), as measured in the same manner as in Reference Example 1.

In the same manner as in Reference Example 1, it was confirmed that at least 20 mg of this magnetic carrier could disperse in 1 mL. of an aqueous solution of a sample containing a biological substance, in the above-mentioned dispersion state, not leas than 90 wt% thereof was collected within 3 seconds by a 2000-3000 gauss magnetic force, and it could reversibly bind with at least 0.4 µg of a biological substance per 1 mg of the magnetic carrier.

### Comparative Reference Example 2

In the same manner as in Reference Example 1 except that air was used as an atmosphere gas during heat treatment, a magnetic carrier was prepared. The obtained magnetic carrier was spherical and had an average particle size of 5 µm, saturation magnetization of 25.3 A·m²/kg (25.3 emu/g), and a coercive force of 10.76 kA/m (135 oersted), as measured in the same manner as in Example 6. By the heat treatment, the magnetite particle was oxidized and converted to maghemite (γ-Fe₂O₃) (confirmed from the position of diffraction peak by X-ray diffraction-method).

### Comparative Reference Example 3

A commercially available dry maghemite (γ-Fe₂O₃) particle (TODA KOGYO CORP.) was used to prepare a magnetic carrier. Such maghemite particle had a saturation magnetization of 74.2 A·m²/kg (74.2 emu/g), a coercive force of 12.75 kA/m (160 oersted) and an average particle size of 0.21 µm. Pure water was added to the above-mentioned, maghemite particle to the total weight of 468 g to give a maghemite particle suspension.

In the same manner as in Reference Example 1 except that 70 g of sodium silicate was added and an ultrasonic dispersion apparatus was used for dispersion and a sodium silicate solution in which maghemite particles were dispersed was prepared, a silica coating treatment and a heat treatment were applied to give a magnetic carrier. The obtained magnetic carrier was spherical and had an average particle size of 5 µm, saturation magnetization of 34.0 A-m²/kg (34.0 emu/g), and a coercive force of 12,75 kA/m (160 oersted), as measured in the same manner as in Reference Example 1.

### Comparative Reference Example 4

In the same manner as in Reference Example 1 except that the amount of sodium silicate to be dissolve in magnetite particle dispersion was set to 120 g during silica coating treatment, a magnetic carrier was prepared. The obtained magnetic carrier was spherical and had an average particle size of 6 µm, saturation magnetization of 29.8 A·m²/kg (29.8 emu/g), and a coercive force of 4 54 kA/m (57 oersted), as measured in the same manner as in Reference Example 1.

### Comparative Reference Example 5

In the same manner as in Comparative Reference Example 4 except that a heat treatment step was not applied, a magnetic carrier was prepared. The obtained magnetic carrier was spherical and had an average particle size of 6 µm, saturation magnetization of 20.4 A·m²/kg (20.4 emu/g), and a coercive force of 2.39 kA/m (90 oersted), as measured in the same manner as in Reference Example 1.

The major preparation conditions of the magnetic carriers obtained in the above-mentioned Reference Examples 1-12 and Comparative Reference Examples 2-5 are shown in Table 3 and main properties of the obtained magnetic carriers are shown in Table 4.

**Table 3**

| | kind | average particle size (µm) | initial state | silica content (wt%) | heat treatment conditions |
|---|---|---|---|---|---|
| Reference Example 1 | magnetite | 0.28 | suspension | 90 | in nitrogen gas, 600°C, 2 hrs. |
| Reference Example 2 | magnetite | 0.13 | suspension | 90 | in nitrogen gas, 600°C, 2 hrs. |
| Reference Example 3 | magnetite | 0.42 | suspension | 90 | in nitrogen gas, 600°C, 2 hrs. |
| Reference Example 5 | magnetite | 0.28 | suspension | 75 | in nitrogen gas, 600°C, 2 hrs. |
| Reference Example 5 | magnetite | 0.28 | suspension | 115 | in nitrogen gas, 600°C, 2 hrs. |
| Reference Example 6 | magnetite | 0.28 | suspension | 90 | in nitrogen gas, 800°C, 2 hrs. |
| Reference Example 7 | magnetite | 0.28 | suspension | 90 | in nitrogen gas, 400°C, 2 hrs. |
| Reference Example 8 | magnetite | 0.28 | suspension | 90 | in argon gas, 600°C, 2 hrs. |
| Reference Example 9 | magnetite | 0.28 | suspension | 90 | in hydrogen gas, 300°C, 2 hrs. |
| Reference Example 10 | magnetite | 0.26 | dry | 90 | in nitrogen gas, 600°C, 2 hrs. |
| Reference Example 11 | magnetite | 0.28 | suspension | 90 | no heat treatment |
| Reference Example 12 | magnetite | 0.28 | suspension | 115 | no heat treatment |
| Comp. Reference Example 2 | magnetite | 0.26 | dry | 90 | in air, 600°C, 2 hrs. |
| Comp. Reference Example 3 | maghemite | 0.21 | dry | 90 | in nitrogen gas, 600°C, 2 hrs. |
| Comp. Reference Example 4 | magnetite | 0.28 | suspension | 155 | in nitrogen gas, 600°C, 2 hrs. |
| Comp. Reference Example 5 | magnetite | 0.28 | suspension | 155 | no heat treatment |

**Table 4**

| | saturation magnetization (A·m²/kg) | coercive force (kA/m) | average particle size (µm) |
|---|---|---|---|
| Reference Example 1 | 47.1 | 5.18 | 5 |
| Reference Example 2 | 45.3 | 6.53 | 4 |
| Reference Example 3 | 47.5 | 3.66 | 7 |
| Reference Example 4 | 51.2 | 5.42 | 4 |
| Reference Example 5 | 40.3 | 4.86 | 6 |
| Reference Example 6 | 47.8 | 2.63 | 5 |
| Reference Example 7 | 43.0 | 8.37 | 5 |
| Reference Example 8 | 47.9 | 5.34 | 5 |
| Reference Example 9 | 50.3 | 4.06 | 5 |
| Reference Example 11 | 40.1 | 9.56 | 6 |
| Reference Example 10 | 38.8 | 7.81 | 5 |
| Reference Example 12 | 32.8 | 8.76 | 6 |
| Comparative Reference Example 2 | 25.3 | 12.76 | 5 |
| Comparative Reference Example 3 | 34.0 | 12.75 | 5 |
| Comparative Reference Example 4 | 25.0 | 4.54 | 6 |
| Comparative Reference Example 5 | 20.4 | 2.39 | 6 |

Then, using the respective magnetic carriers for binding with nucleic acid obtained in the above-mentioned Reference Examples 1-12 and Comparative Reference Examples 2-5, the nucleic acid was extracted and recovered from the biological samples according to the aforementioned extraction test, and the recovery performance was examined. The results are shown in Table 5.

**Table 5**

| | recovery amount of (ng) nucleic acid |
|---|---|
| Reference Example 1 | 1920 |
| Reference Example 2 | 1900 |
| Reference Example 3 | 1850 |
| Reference Example 4 | 1820 |
| Reference Example 5 | 1800 |
| Reference Example 6 | 1900 |
| Reference Example 7 | 1780 |
| Reference Example 8 | 1910 |
| Reference Example 9 | 1900 |
| Reference Example 10 | 1560 |
| Reference Example 11 | 1580 |
| Reference Example 12 | 1470 |
| Comparative Reference Example 2 | 1010 |
| Comparative Reference Example 3 | 1190 |
| Comparative Reference Example 4 | 1080 |
| Comparative Reference Example 5 | 935 |

From the above-mentioned results, it is clear that the magnetic carrier comprising a ferromagnetic iron oxide particle and silica coating the surface of the ferromagnetic iron oxide particle and having a saturation magnetization of 30-80 A·m²/kg, a coercive force of 2.39-11.94 kA/m and an average particle size of 0.1-10 µm can disperse in an amount of at least 20 mg in 1 mL of an aqueous solution of a sample containing a biological substance, can be collected by not less than 90 wt% within 3 seconds in the presence of a magnetic field of 2000-3000 gauss, and can reversibly bind with at least 0.4 µg of a biological substance per 1 mg thereof, with which carrier, an isolation method of biological substances having strikingly improved isolation and purification efficiency of the biological substance as compared to conventional methods has been established. This is because the use of a magnetic carrier having the above-mentioned particular properties has made the bindability with nucleic acid/collectability of magnetic carrier by magnetic field and dispersibility of magnetic carrier/elution property of nucleic acid compatible.

### Reference Example 13

### <Synthesis of magnetite particle>

Ferrous sulfate (100 g, FeSO₄ 7H₂ O) was dissolved in 1,000 cc of pure water. Sodium hydroxide (28.8 g, equimolar amount to ferrous sulfate) was dissolved in 500 cc of pure water, while stirring the aqueous ferrous sulfate solution, an aqueous sodium hydroxide solution was dropwise added over 1 hr. to give ferrous hydroxide precipitate. After the completion of the dropwise addition, the temperature of the suspension containing the ferrous hydroxide precipitate was raised to 75°C with stirring and oxidation was conducted while blowing in air using an air pump at a flow rate of 250 liter/hr. for 8 hrs. to give a magnetite particle. This particle was nearly spherical and had an average particle size of about 0.22 µm.

The above-mentioned average particle size was determined by measuring the size of 300 particles on a transmission electron microscopic photograph and calculating a number average thereof.

### <Adhesion of compound comprising silicon and aluminum>

After thorough washing of the magnetite particle suspension obtained as above with pure water, the weight of the magnetite particle and water was adjusted to 10 g and 200 g, respectively, without drying. The amount of the magnetite in the suspension after water washing was determined by sampling and drying a part thereof.

Separately from the suspension of magnetite particle, sodium silicate (2 g) was dissolved in pure water (10 g). Aluminum chloride (0.5 g) was dissolved in 1N aqueous sodium hydroxide solution (10 g). The both solutions were mixed to give a mixed solution of sodium silicate and aluminum chloride, which was added to the above-mentioned magnetite particle suspension, and an aqueous hydrochloric acid solution diluted with pure water was dropwise added with stirring over about 1 hr. for neutralization to near neutral. After the completion of the dropwise addition, the mixture was stirred for one more hour.

When the amount of the magnetite particle relative to water is adjusted to 1-10 wt%, a compound comprising silicon and aluminum can be preferentially adhered near the surface of the magnetite particle. The suspension thus treated was filtrated and dried in air at 90°C. The particle after drying was pulverized and subjected to heat treatment in a nitrogen gas at 200°C for 2 hrs. The particle after heat treatment was washed with pure water to remove unreacted materials and reaction product precipitated in parts other than the magnetite particle surface.

The thus-obtained magnetic carrier for nucleic acid was spherical or granular and had an average particle size of 0.29 µm, a coercive force of 5.58 kA/m (70 oersted) and a saturation magnetization of 67.9 A·m²/kg (67.9 emu/g). The amount of aluminum in the mixed oxide of the adhered silicon and aluminum was 8.9 wt% of the total amount of silicon and aluminum. The content of silicon and aluminum was 23.5 wt% of the magnetite particle upon conversion to silica (SiO₂) and alumina (Al₂O₃) contents.

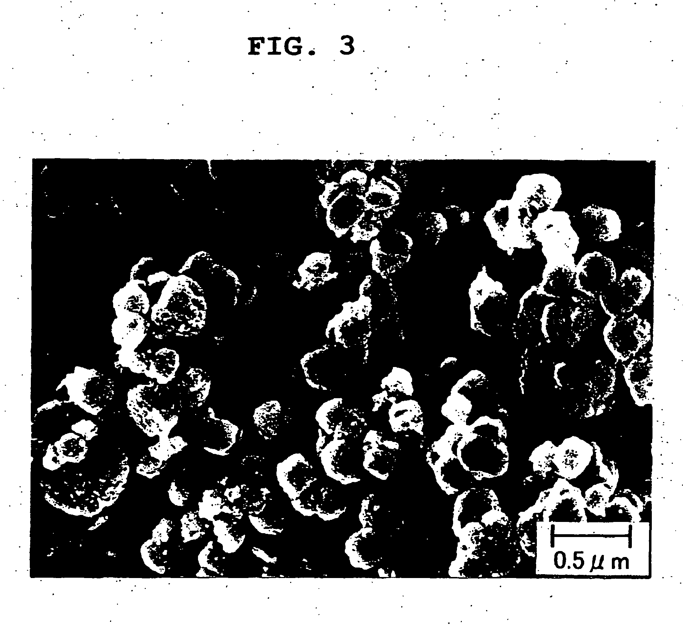

Fig. 3 shows an SEM image of the magnetic carrier. This image clearly shows respective magnetite particles, no precipitate is found other than on magnetite particles, and a mixed oxide of silicon and aluminum is adhered near the surface of the magnetite particle.

Then, this magnetic carrier (0.5 g) was placed in a 10 mm diameter columnar glass tube, pure water (1.5 g) was added, and the mixture was dispersed for 30 min. using an ultrasonic dispersion apparatus. Thereafter, the mixture was taken out from the ultrasonic dispersion apparatus, stood still for 15 min. and the volume of the sedimentation of the magnetic carrier was measured. The volume of the sedimentation of magnetic carrier was 860 mm³ (height 11 mm).

### Reference Example 14

In the same manner as in Reference Example 13 except that, in the adhesion step of a compound comprising silicon and aluminum, the amount of sodium silicate was changed from 2 g to 1.8 g and the amount of aluminum chloride was changed from 0.5 g to 1 g, a compound comprising silicon and aluminum was adhered to the magnetite particle and subsequent heat treatment was applied in the same manner as in Reference Example 13, a magnetic: carrier for nucleic acid was obtained.

This magnetic carrier was spherical or granular and had an average particle size of 0.31 µm, a, coercive force of 5.98 kA/m (75 oersted) and a saturation magnetization of 66.8 A-m²/kg. (66.8 emu/g). The amount of aluminum in the mixed oxide of the adhered silicon and aluminum was 17.5 wt% of the total amount of silicon and aluminum. The content of silicon and aluminum was 24.8 wt% of the magnetite particle upon conversion to silica (SiO₂) and alumina (Al₂O₃) contents.

From the SEM observation, adhesion of mixed oxide of silicon and aluminum near the surface of each magnetite particle in this magnetic carrier was clearly confirmed. This magnetic carrier was measured for sedimentation volume in the same manner as in Reference Example 13 and found to be 903 mm³ (hL-i*ght 11.5mm).

### Reference Example 15

In the same manner as in Reference Example 13 except that, in the adhesion step of a compound comprising silicon and aluminum, the amount of sodium silicate was changed from 2 g to 2.2 g and the amount of aluminum chloride was changed from 0.5 g to 0.3 g, a compound comprising silicon and aluminum was adhered to the magnetite particle and subsequent heat treatment was applied in the same manner as in Example 1, a magnetic carrier for nucleic acid was obtained.

This magnetic carrier was spherical or granular and had an average particle size of 0.30 µm, a coercive force of 5.18 kA/m (65 oersted) and a saturation magnetization of 69.0 A·m²/kg (69.0 emu/g) . The amount of aluminum in the mixed oxide of the adhered silicon and aluminum was 4.5 wt% of the total amount of silicon and aluminum. The content of silicon and aluminum was 22.0 wt% of the magnetite particle upon conversion to silica (SiO₂) and alumina (Al₂O₃) contents.

From the SEM observation, adhesion of mixed oxide of silicon and aluminum near the surface of each magnetite particle in this magnetic carrier was clearly confirmed. This magnetic carrier was measured for sedimentation volume in the same manner as in Reference Example 13 found to be 942 mm³ (height 12 mm).

### Comparative Reference Example 6

In the same manner as in Reference Example 13 except that, in the adhesion step of a compound comprising silicon and aluminum, the amount of sodium silicate was changed from 2 g to 2.3 g and the amount of aluminum chloride was changed from 0.5 g to 0 g, silicon compound alone was adhered to the magnetite particle and subsequent heat treatment was applied in the same manner as in Reference Example 13, a magnetic carrier for nucleic acid was obtained.

This magnetic carrier was spherical or granular and had an average particle size of 0.29 µm, a coercive force of 5.58 kA/m (70 oersted) and a saturation magnetization of 68.1 A·m²/kg (68.1 emu/g). The silicon content was 23.1 wt% of the magnetite particle upon conversion to silica (SiO₂) content. This magnetic carrier was measured for sedimentation volume in the same manner as in Reference Example 13 and found to be 1178 mm³ (height 15 mm).

With regard to the respective magnetic carriers for binding with nucleic acid, which were obtained in the above-mentioned Reference Examples 13-15 and Comparative Reference Example 6, the average particle size, the ratio of aluminum in the total amount of silicon and aluminum [Al/(Si+Al)] and the ratio of silica and alumina relative to ferromagnetic iron oxide particle [(SiO₂+Al₂O₃)/Fe₃O₄] are collectively shown in Table 6. With regard to the respective magnetic carriers for binding with nucleic acid, which were obtained in the above-mentioned Reference Examples 13-15 and Comparative Reference Example 6, the coercive force, saturation magnetization and sedimentation volume are collectively shown in Table 7.

**Table 6**

| | average particle size (µm) | Al/(Si+Al) (wt%) | (SiO₂+Al₂O₃)/(Fe₃O₄) (wt%) |
|---|---|---|---|
| Reference Example 13 | 0.29 | 8.9 | 23.5 |
| Reference Example 14 | 0.31 | 17.5 | 24.8 |
| Reference Example 15 | 0.30 | 4.5 | 22.0 |
| Comparative Reference Example 6 | 0.29 | 0 | 23.1 |

**Table 7**

| | coercive force (kA/m) | saturation magnetization (A·m²/kg) | sedimentation volume (mm³) |
|---|---|---|---|
| Reference Example 13 | 5.58 | 67.9 | 860 |
| Reference Example 14 | 5.98 | 66.8 | 903 |
| Reference Example 15 | 5.18 | 69.0 | 942 |
| Comparative Reference Example 6 | 5.58 | 68.1 | 1178 |

With regard to the respective magnetic carriers for binding with nucleic acid, which were obtained in the above-mentioned Reference Examples 13-15 and Comparative Reference Example 6, the nucleic acid was extracted and recovered from the biological samples according to the aforementioned extraction test, and the recovery performance was examined. The results are shown in Table 8.

**Table 8**

| | recovery amount of nucleic acid (ng) |
|---|---|
| Reference Example 13 | 1920 |
| Reference Example 14 | 1930 |
| Reference Example 15 | 1920 |
| Comparative Reference Example 6 | 1910 |

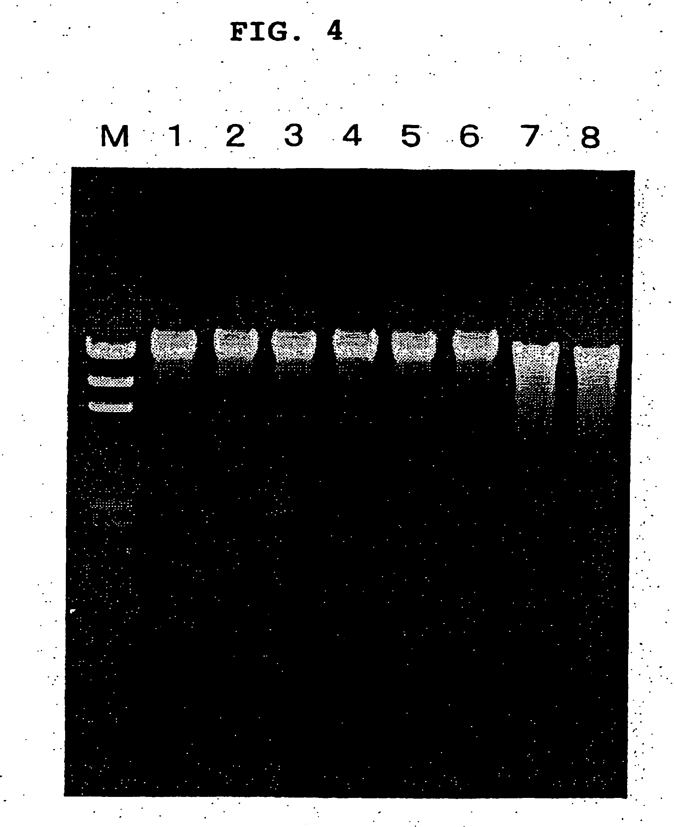

Then, the nucleic acid solution (5 µl) recovered from the above-mentioned extraction test was electrophoresed on 1% agarose gel, stained with ethidium bromide and fluorescence upon ultraviolet radiation was detected. The results are as shown in Fig. 4. The electrophoresis conditions were constant voltage 100 V, 30 min. The method of operation and other conditions followed the technique described in Maniatis et al., "Molecular Cloning" (1982). In addition to the nucleic acid solution, a molecular weight marker was also simultaneously electrophoresed and used as an index for comparison of the chain length of the detected nucleic acid.

In the electrophoretic image shown in Fig. 4, "M" means the above-mentioned molecular weight marker, "lanes 1, 2" show the results (2 samples) of extraction and purification of nucleic acid by the use of the carrier of Reference Example 13, "lanes 3, 4" show the results (2 samples) of extraction and purification of nucleic acid by the use of the carrier of Reference Example 14, "lanes 5, 6" show the results (2 samples) of extraction and purification of nucleic acid by the use of the carrier of Reference Example 15, and "lanes 7, 8" show the results. (2 samples) of extraction and purification of nucleic acid by the use of the carrier of Comparative Reference Example 6.

As is clear from the above-mentioned results in Tables 6-8, the magnetic carriers of Reference Examples 14-15, wherein a compound comprising silicon and aluminum was adhered near the surface of the magnetite particle showed almost the same amount of nucleic acid extraction, clearly small sedimentation volume, fine surface smoothness and fine flowability, were by no means inferior in the extraction performance of nucleic acid, and were superior in adhesion uniformity.

Another experiment demonstrating the flowability was performed as follows.

That is, a given amounts of the magnetic carriers for binding with nucleic acid of Reference Examples 13-15 and Comparative Reference Example 6 was dispersed in water, and a given amount of the aqueous suspension was added dropwise on a glass plate. Then, this aqueous suspension was linearly moved using a magnet from the back of the glass plate. After moving a certain distance, the weight of the magnetic carrier moved with the aqueous suspension was measured.

As a result, the magnetic carrier that moved with the suspension clearly weighed heavier in Reference Examples 13-15 than in Comparative Reference Example 6. This means that the magnetic carriers of Reference Example 13-15 are superior to the magnetic carrier of Comparative Reference Example 6 in the adhesion uniformity of the compound (oxide) adhered near the surface of the magnetite particle and are more superior in the flowability.

As is clear from the above-mentioned results shown in Fig. 4, the nucleic acid recovered using the magnetic carriers of Reference Examples 13-15 shows smaller molecular weight distribution than the nucleic acid recovered using the magnetic carrier of Comparative Reference Example 6. The reason therefor is not clear but it is postulated that adhesion of a compound comprising silicon and aluminum to the magnetite particle makes the particle surface of the magnetic carrier smooth, which resists cleavage of the nucleic acid. In contrast, a magnetic carrier, to which silicon compound alone is adhered, shows inferior adhesion uniformity. It is postulated, therefore, that the particle surface of the magnetic carrier has concaves and convexes to facilitate cleavage of nucleic acid, which in turn makes broad molecular weight distribution of the nucleic acid.

As explained above, the magnetic carrier of the present invention is superior in dispersibility in aqueous solution, collectability by a magnetic field, reversible binding ability with a biological substance, elution property of the bound biological substance, and isolation and purification efficiency of biological substance., as compared to conventional carriers.

## Claims

1. A magnetic carrier for a nucleic acid, which has a saturation magnetization of 30-80 A-m²/kg and a coercive force of 2.39-11.94 kA/m, the magnetic carrier comprising magnetite particles and a silica coating on the surface of said magnetite particles, wherein the amount of the silica coating is 3-100wt% of the magnetite particles, and the magnetic carrier has an average particle size of 0.12-0.45µm.

2. Use of the magnetic carrier of claim 1 for binding a nucleic acid by bringing the carrier into contact with the nucleic acid in an aqueous solution of a sample containing the nucleic acid.

3. A method of isolating a nucleic acid, which comprises
forming a complex of a nucleic acid and a magnetic carrier by bringing the magnetic carrier of claim 1 into contact with said nucleic acid in an aqueous solution of a sample containing the nucleic acid,
separating the complex from the sample by an external magnetic field, and
eluting the nucleic acid from the complex.

4. A production method of the magnetic carrier of claim 1, which comprises the steps of: preparing an aqueous suspension comprising 100 parts by weight of water and 1-10 parts by weight of magnetite particles having an aspect ratio fo 1.0-1.2 suspended in said water, b) adding 0.3-2 parts by weight of sodium silicate on conversion to SiO₂ to said aqueous suspension, and c) neutralizing soldium silicate by adding an acid to allow precipitation of silica.

5. The production method of claim 4, further comprising a heat treatment of the carrier in an inert gas.

6. The production method of claim 4 or 5, comprising subjecting magnetite particles coated with silica to a heat treatment at 200-800°C.

7. The production method of claim 6, wherein the heat treatment is conducted in an atmospheric gas of an inert, gas or a reducing gas.

8. The production method of claim 6 or 7, wherein the magnetite particles are synthesized by oxidation in an aqueous solution and applied to a silica coating treatment without drying.

## Patentansprüche

1. Magnetischer Träger für eine Nukleinsäure, der eine Sättigungsmagnetisierung von 30 bis 80 A·m²/kg und eine Koerzitivkraft von 2,39 bis 11,94 kA/m hat, wobei der magnetische Träger Magnetitteilchen und eine Silikabeschichtung auf der Oberfläche der Magnetitteilchen aufweist, wobei die Menge der Silikabeschichtung 3 bis 100 Gew.-% der Magnetitteilchen beträgt und der magnetische Träger eine mittlere Teilchengröße von 0,12 bis 0,45 µm hat.

2. Verwendung des magnetischen Trägers nach Anspruch 1 zur Bindung einer Nukleinsäure durch In-Kontakt-bringen des Trägers mit der Nukleinsäure in einer wäßrigen Lösung einer die Nukleinsäure enthaltenden Probe.

3. Verfahren zum Isolieren einer Nukleinsäure, das aufweist:
Bilden eines Komplexes aus einer Nukleinsäure und einem magnetischen Träger durch In-Kontakt-bringen des magnetischen Trägers nach Anspruch 1 mit der Nukleinsäure in einer wäßrigen Lösung einer die Nukleinsäure enthaltenden Probe,
Trennen des Komplexes von der Probe durch ein externes Magnetfeld, und
Eluieren der Nukleinsäure aus dem Komplex.

4. Herstellungsverfahren des magnetischen Trägers nach Anspruch 1, das die folgenden Schritte aufweist:
a) Herstellen einer wäßrigen Lösung mit 100 Gewichtsteilen Wasser und 1 bis 10 Gewichtsteilen Magnetitteilchen mit einem Aspektverhältnis von 1,0 bis 1,2, die im Wasser suspendiert sind,
b) Zugeben von 0,3 bis 2 Gewichtsteilen Natriumsilicat bei Umwandlung zu SiO₂ zur wäßrigen Suspension, und
c) Neutralisieren von Natriumsilicat durch Zugeben einer Säure, damit Silika ausscheiden kann.

5. Herstellungsverfahren nach Anspruch 4, ferner mit einer Wärmebehandlung des Trägers in einem Inertgas.

6. Herstellungsverfahren nach Anspruch 4 oder 5, ferner mit Einwirkenlassen einer Wärmebehandlung bei 200 bis 800 °C auf Magnetitteilchen, die mit Silika beschichtet sind.

7. Herstellungsverfahren nach Anspruch 6, wobei die Wärmebehandlung in einem Atmosphärengas aus einem Inertgas oder einem Reduktionsgas durchgeführt wird.

8. Herstellungsverfahren nach Anspruch 6 oder 7, wobei die Magnetitteilchen durch Oxidation in einer wäßrigen Lösung synthetisiert und Silikabeschichtungsbehandlung ohne Trocknen ausgesetzt werden.

## Revendications

1. Support magnétique destiné à un acide nucléique, qui possède une magnétisation à saturation de 30 à 80 A·m²/kg et une force coercitive de 2,39 à 11,94 kA/m, le support magnétique comprenant des particules de magnétite et un revêtement de silice sur la surface desdites particules de magnétite, dans lequel la quantité de revêtement de silice est de 3 à 100% en poids des particules de magnétite, et le support magnétique possède une taille de particules moyenne de 0,12 à 0,45 µm.

2. Utilisation du support magnétique selon la revendication 1 pour lier un acide nucléique en amenant le support en contact avec l'acide nucléique dans une solution aqueuse d'un échantillon contenant l'acide nucléique.

3. Procédé d'isolation d'un acide nucléique, qui comprend la formation d'un complexe d'un acide nucléique et d'un support magnétique en amenant le support magnétique selon la revendication 1 en contact avec ledit acide nucléique dans une solution aqueuse de l'échantillon contenant l'acide nucléique,
la séparation du complexe de l'échantillon par un champ magnétique externe, et
l'élution de l'acide nucléique à partir du complexe.

4. Procédé de production du support magnétique selon la revendication 1, qui comprend les étapes consistant à :
a) préparer une suspension aqueuse comprenant 100 parties en poids d'eau et 1 à 10 parties en poids de particules de magnétite ayant un rapport d'aspect de 1,0 à 1,2 mises en suspension dans ladite eau,
b) ajouter 0,3 à 2 parties en poids de silicate de sodium, lors de la conversion en SiO₂, à ladite eau aqueuse, et
c) neutraliser le silicate de sodium en ajoutant un acide afin de permettre une précipitation de la silice.

5. Procédé de production selon la revendication 4, comprenant en outre un traitement thermique du support dans un gaz inerte.

6. Procédé de production selon la revendication 4, ou 5, comprenant le fait de soumettre des particules de magnétite recouverte de silice à un traitement thermique à 200 à 800°C.

7. Procédé de production selon la revendication 6, dans lequel le traitement thermique est effectué dans une atmosphère gazeuse d'un gaz inerte ou d'un gaz réducteur.

8. Procédé de production selon la revendication 6 ou 7, dans lequel les particules de magnétite sont synthétisées par oxydation dans une solution aqueuse et appliquées à un traitement de revêtement de silice sans séchage.
